# FASCICULE DE BREVET EUROPEEN

(11) **EP 2 859 107 B1**
(45) Date de publication et mention de la délivrance du brevet: **19.08.2020**
(21) Numéro de dépôt: 13731390.4
(22) Date de dépôt: 07.06.2013
(51) Int. Cl.: C12P 7/10, C12P 19/02, C12P 19/14, C13K 1/02

(54) **PROCÉDÉ DE TRAITEMENT ENZYMATIQUE D'UNE MATIÈRE LIGNO-CELLULOSIQUE SOLIDE**
VERFAHREN ZUR ENZYMATISCHEN BEHANDLUNG VON FESTEN LIGNOCELLULOSEMATERIALIEN
METHOD OF ENZYMATIC TREATMENT OF A SOLID LIGNOCELLULOSIC MATERIAL

(30) Priorité: 08.06.2012 FR 1255394
(43) Date de publication de la demande: 15.04.2015
(73) Titulaire: Institut National Polytechnique de Toulouse, 31400 Toulouse (FR); Centro de Investigaciones Energéticas, Medioambientales y Tecnológicas, 28040 Madrid (ES); UNIVERSIDAD NACIONAL AUTONOMA DE MEXICO, Mexico, DF 04510 (MX)
(72) Inventeur: VILAREM, Gérard, F-31130 Balma (FR); RIGAL, Luc, F-31240 Saint-Jean (FR); VANDENBOSSCHE, Virginie, F-31570 Sainte Foy d'Aigrefeuille (FR); BRAULT, Julien, F-95290 L'Isle Adam (FR); HERNANDEZ LUNA, Martin, 14020 Mexico D.F. (MX); HERNANDEZ MELENDEZ, Oscar, 08840 Mexico D.F. (MX); VIVALDO LIMA, Eduardo, 03600 Mexico D.F. (MX); BARZANA GARCIA, Eduardo, Mexico, 11000 (MX); BALLESTEROS, Mercedes, E-28035 Madrid (ES); DUQUE, Aleta, E-28094 Madrid (ES); MANZANARES, Paloma, 28035 Madrid (ES); SIIKA-AHO, Matti Samuel, FI-00810 Helsinki (FI); UUSITALO, Jaana Maria, FI-02360 Espoo (FI); MATA-SEGREDA, Julio, Cedros De Montes De Oca 2050 (CR); GUILLOUET, Stéphane, F-31290 Vallegue (FR); LOMBARD, Eric, F-31330 Grenade (FR); CAMELEYRE, Xavier, F-31320 Castanet Tolosan (FR)
(74) Mandataire: Cabinet BARRE LAFORGUE & associés
(86) Numéro de dépôt international: PCT/FR2013/051317
(87) Numéro de publication internationale: WO 2013/182827

(56) Documents cités:
- WO-A1-2009/124072
- WO-A1-2010/063980
- WO-A1-2011/021272
- US-A1- 2010 317 053
- US-A1- 2012 125 549
- OGIER J-C ET AL: "PRODUCTION D'ETHANOL A PARTIR DE BIOMASSE LIGNOCELLULOSIQUE", OIL & GAS SCIENCE & TECHNOLOGY: REVUE DE L'INSTITUT FRANCAIS DU PETROLE, EDITIONS TECHNIP. PARIS, FR, vol. 54, no. 1, 1 janvier 1999 (1999-01-01), pages 67-94, XP000831737, ISSN: 1294-4475
- JOSEPH R SAMANIUK ET AL: "The effect of high intensity mixing on the enzymatic hydrolysis of concentrated cellulose fiber suspensions", BIORESOURCE TECHNOLOGY, ELSEVIER BV, GB, vol. 102, no. 6, 25 novembre 2010 (2010-11-25), pages 4489-4494, XP028361178, ISSN: 0960-8524, DOI: 10.1016/J.BIORTECH.2010.11.117 [extrait le 2010-12-03]
- SHUJING ZHANG ET AL: "Pretreatment of Corn Stover with Twin-Screw Extrusion Followed by Enzymatic Saccharification", APPLIED BIOCHEMISTRY AND BIOTECHNOLOGY ; PART A: ENZYME ENGINEERING AND BIOTECHNOLOGY, HUMANA PRESS INC, NEW YORK, vol. 166, no. 2, 6 décembre 2011 (2011-12-06), pages 458-469, XP035002247, ISSN: 1559-0291, DOI: 10.1007/S12010-011-9441-6

## Description

L'invention concerne un procédé de traitement enzymatique d'une matière ligno-cellulosique solide. L'invention concerne un tel procédé pour la préparation d'une solution -notamment une solution aqueuse- de saccharides et/ou de composés phénoliques. L'invention concerne aussi l'utilisation d'un tel procédé pour la préparation d'une telle solution aqueuse de saccharides en vue de leur fermentation -notamment alcoolique- pour la production d'éthanol.

En particulier, l'invention vise un procédé pour la préparation d'une solution de saccharides comprenant au moins un monosaccharide et/ou au moins un oligosaccharide et/ou au moins un polysaccharide de degré de polymérisation et/ou de masse moléculaire apte à former une solution.

Un tel procédé de traitement enzymatique d'une matière ligno-cellulosique solide trouve ses applications dans le domaine général de la valorisation de ressources végétales, telles que des déchets végétaux -en particulier des déchets végétaux issus de l'agriculture ou de l'industrie agroalimentaire-, ou telles que des cultures de plantes herbacées destinées à la production de biomasse -notamment pour la production d'énergie-. Un tel procédé trouve aussi ses applications dans la transformation de déchets végétaux en matériaux qui ne sont plus des déchets et qui peuvent être transformés, par exemple, en éthanol par fermentation alcoolique.

On connaît (US 2007/0031918) un procédé de traitement de biomasse pour la production de sucres fermentables dans lequel on traite ladite biomasse par une solution aqueuse d'ammoniaque et, le cas échéant, d'une base de façon à former une dispersion concentrée de la biomasse dans l'ammoniaque, puis on ajuste le pH de la dispersion de biomasse à une valeur de pH 5 et on hydrolyse par voie enzymatique les sucres de la biomasse au moyen d'une cellulase pendant une durée de l'ordre de 96 heures.

Un tel procédé nécessite une étape de mise de la biomasse sous pression réduite en vue de l'élimination de l'ammoniaque du milieu réactionnel avant l'addition des cellulases. Une telle étape est complexe à mettre en œuvre à l'échelle industrielle. Un tel procédé ne permet pas non plus de traiter rapidement une composition de biomasse, notamment en moins de 96 heures.

On connait aussi de Samaniuk et al. (2011), « Bioressource technology, 102, 4489-4494 », un procédé dans lequel on soumet des tiges de maïs broyées à un traitement en milieu acide puis à un traitement de dégradation enzymatique de la matière solide obtenue par ce traitement en milieu acide. Un tel procédé nécessite une étape préalable de broyage de tiges de maïs, une étape de neutralisation du milieu acide, une étape de filtration/lavage sous vide du milieu neutralisé adaptée pour préparer une matière solide préalablement à son traitement enzymatique. Un tel procédé est complexe et n'est pas adapté pour une mise en œuvre à l'échelle industrielle.

Le document WO 2011/021272 décrit un procédé de prétraitement d'une biomasse végétale dans une extrudeuse.

L'invention vise à pallier les inconvénients précédemment évoqués en proposant un procédé de traitement enzymatique d'une matière ligno-cellulosique solide pour la fabrication d'une solution -notamment d'une solution aqueuse- de saccharides et/ou de composés phénoliques qui soit rapide à mettre en œuvre. En particulier, un tel procédé permet d'obtenir une solution de saccharides et/ou de composés phénoliques à partir d'une matière ligno-cellulosique solide en moins d'une heure -notamment dans une durée de l'ordre de 10 min-.

L'invention vise également à proposer un procédé de traitement enzymatique d'une matière ligno-cellulosique solide adapté pour permettre la valorisation de ressources végétales (biomasse) qui ne sont plus des déchets mais qui sont susceptibles d'être valorisées notamment sous la forme de composés phénoliques, de saccharides issus des hémicelluloses et de la cellulose de la matière ligno-cellulosique solide, par exemple sous la forme de monosaccharide(s) tel que le glucose ou sous la forme de ses produits de fermentation -par exemple sous la forme d'éthanol-.

L'invention vise donc en particulier mais non exclusivement un procédé de traitement enzymatique d'une matière ligno-cellulosique solide en vue de la production d'éthanol et de son utilisation comme biocarburant.

En particulier, l'invention vise à proposer un procédé de traitement enzymatique d'une matière ligno-cellulosique solide qui est disponible en grande quantité.

Un autre objectif de l'invention est de proposer un tel procédé de traitement enzymatique d'une matière ligno-cellulosique solide qui ne nécessite pas un raffinage de la cellulose produite à partir d'une ressource végétale avant sa transformation en saccharides solubilisés et en glucose en vue de sa fermentation alcoolique.

L'invention vise aussi un tel procédé de traitement enzymatique d'une matière ligno-cellulosique solide dans lequel on ne réalise aucune étape d'hydrolyse acide -notamment à chaud- susceptible de générer des sous-produits de réaction aptes à interférer avec -voire à inhiber- une étape subséquente de fermentation alcoolique de glucose.

L'invention vise également à atteindre tous ces objectifs à moindre coût, en proposant un tel procédé de traitement enzymatique d'une matière ligno-cellulosique solide qui soit simple dans sa mise en oeuvre et qui ne nécessite aucune modification des installations industrielles préexistantes.

L'invention vise aussi un tel procédé de traitement enzymatique d'une matière ligno-cellulosique solide adapté pour permettre une préparation rapide d'une solution de saccharides et/ou de composés phénoliques.

L'invention vise aussi un tel procédé de traitement enzymatique -notamment de dégradation enzymatique- d'une matière ligno-cellulosique solide d'efficacité améliorée.

Pour ce faire, l'invention concerne un procédé de traitement d'une matière ligno-cellulosique solide selon la revendication 1.

Dans toute la suite l'expression "matière ligno-cellulosique" désigne toute matière naturelle comprenant de la cellulose, de l'hémicellulose ou des lignines. En particulier, une telle matière ligno-cellulosique solide selon la présente invention comprend une proportion massique de cellulose comprise entre 20 % et 100 % de la matière sèche. Il peut donc s'agir de cellulose pure. Le traitement selon l'invention d'une telle cellulose pure permet de former une solution de saccharides et le cas échéant de glucose.

Un tel procédé de traitement mécano-enzymatique d'une matière ligno-cellulosique solide est adapté pour permettre la fabrication d'une solution aqueuse d'au moins un produit de dégradation enzymatique de l'un des constituants de la matière ligno-cellulosique solide. En particulier, un tel procédé est adapté pour permettre la fabrication d'une solution aqueuse de saccharides et/ou de D-glucose à partir de ladite matière ligno-cellulosique solide.

Dans un tel procédé de traitement d'une matière ligno-cellulosique solide selon l'invention, on soumet d'abord la matière ligno-cellulosique solide à un traitement, dit traitement mécano-chimique :
- de malaxage de ladite matière ligno-cellulosique solide -notamment dans un réacteur de malaxage adapté pour permettre une succession de phases de compression, de détente et de cisaillement mécaniques de la matière ligno-cellulosique solide, et ;
- de dégradation chimique de ladite matière ligno-cellulosique solide ;
dans lequel on met la matière ligno-cellulosique solide en contact avec une solution alcaline de façon à former une composition intermédiaire présentant une matière ligno-cellulosique, dite matière ligno-cellulosique hydratée, de digestibilité enzymatique -notamment de la cellulose- augmentée par rapport à la digestibilité -notamment de la cellulose- de la matière ligno-cellulosique solide de départ.

Avantageusement, dans un procédé selon l'invention on soumet la matière ligno-cellulosique solide à un premier traitement mécano-chimique, de malaxage et de dégradation chimique de ladite matière ligno-cellulosique solide, dans lequel on met la matière ligno-cellulosique solide en contact avec la solution alcaline de façon à former par imprégnation et gonflement de la matière ligno-cellulosique solide, une composition intermédiaire présentant une matière ligno-cellulosique solide, dite matière ligno-cellulosique hydratée, présentant une sensibilité à l'hydrolyse enzymatique augmentée par rapport à la sensibilité à l'hydrolyse enzymatique de la matière ligno-cellulosique solide de départ. On soumet ensuite la matière ligno-cellulosique hydratée, le cas échéant après neutralisation de la composition intermédiaire, au traitement mécano-enzymatique.

On caractérise la matière ligno-cellulosique hydratée par le fait qu'elle présente, en particulier une proportion de cellulose de type II par rapport à la cellulose de type I qui est augmentée par rapport à la proportion de cellulose de type II par rapport à la cellulose de type I de la matière ligno-cellulosique solide de départ. On quantifie la cellulose de type I et de type II par des techniques connues en elles-mêmes de l'homme du métier, en particulier par diffraction des rayons X de la matière ligno-cellulosique solide de départ et de la matière ligno-cellulosique hydratée sèches.

Avantageusement, la solution alcaline est une solution aqueuse d'au moins un hydroxyde d'un métal alcalin -notamment choisi dans le groupe formé de l'hydroxyde de sodium et de l'hydroxyde de potassium-. Avantageusement, le pH de la solution alcaline est supérieur à 7,5. Dans un procédé selon l'invention, le pH de la solution alcaline peut prendre toute valeur, par exemple de l'ordre de 8 ou supérieur à 13.

Avantageusement et selon l'invention, on réalise le traitement mécano-chimique à une température comprise entre 50°C et 150°C.

Dans un tel procédé de traitement d'une matière ligno-cellulosique solide selon l'invention, on forme une dispersion aqueuse concentrée de ladite matière ligno-cellulosique hydratée. Une telle dispersion aqueuse comprend au moins une enzyme de dégradation de ladite matière ligno-cellulosique hydratée qui est adaptée pour permettre une scission moléculaire d'au moins l'un des constituants de la matière ligno-cellulosique hydratée.

En effet, les inventeurs ont constaté que, contrairement au préjugé reconnu dans l'état de la technique, il est possible de réaliser une attaque enzymatique d'une matière ligno-cellulosique hydratée solide sous la forme d'une dispersion aqueuse directement dans un réacteur de malaxage. En particulier, les inventeurs ont observé qu'une telle attaque enzymatique de la matière ligno-cellulosique hydratée solide reste très efficace malgré la concentration élevée de ladite matière ligno-cellulosique hydratée solide dans la dispersion aqueuse, malgré les conditions de compression/détente successives et de cisaillement mécaniques et de température auxquelles est soumise la dispersion aqueuse comprenant l'(les) enzyme(s) de dégradation de ladite matière ligno-cellulosique hydratée solide dans le réacteur de malaxage.

Avantageusement, un tel procédé selon l'invention permet d'obtenir une dégradation enzymatique d'une matière ligno-cellulosique solide dans un réacteur de malaxage pour une durée courte de traitement (en particulier une durée inférieure à 1 heure), alors que les traitements enzymatiques de l'état de la technique nécessitent une durée de traitement supérieure à une heure -notamment supérieure à plusieurs heures-.

En fait, l'invention va à l'encontre de préjugés de l'état de la technique selon lesquels, pour réaliser une digestion enzymatique d'un substrat ;
- il convient de former une solution -et non une dispersion-comprenant l'enzyme et le substrat, dans des conditions de concentration dudit substrat qui soit faible et adaptée pour limiter l'inhibition de l'enzyme par excès de substrat ;
- il convient de ne pas faire subir au milieu comprenant le substrat des contraintes de température et de pression qui sont inhabituelles pour l'utilisation d'enzymes.

Ce faisant, les inventeurs ont observé qu'il est possible de façon surprenante de réaliser une hydrolyse enzymatique -notamment une hydrolyse partielle ou une hydrolyse totale- d'une matière ligno-cellulosique solide -notamment de la cellulose, des lignines et des hémicelluloses de ladite matière ligno-cellulosique solide- dans un milieu concentré et dans des conditions de malaxage -notamment de compression/détente successives et de cisaillement- et de température non décrites dans l'état de la technique.

Dans un procédé selon l'invention, on forme, outre la solution d'hydrolyse, un résidu solide susceptible d'être soumis à un traitement de fermentation apte à produire une quantité d'éthanol, le traitement de fermentation du résidu solide présentant un rendement, exprimé en masse d'éthanol produit lors du traitement de fermentation rapportée à la masse du résidu solide qui est supérieur au rendement de fermentation de la matière ligno-cellulosique solide de départ réalisée dans les mêmes conditions.

Avantageusement et selon l'invention, la dispersion aqueuse présente un rapport entre la masse de composition aqueuse de la dispersion aqueuse et la masse de matière sèche de ladite matière ligno-cellulosique solide de la dispersion aqueuse compris entre 1 et 4.

On détermine la masse de matière sèche de la matière ligno-cellulosique solide par une méthode connue en elle-même de l'homme du métier et dans laquelle on réalise une pesée de ladite matière ligno-cellulosique préalablement exposée à un traitement de séchage à une température de l'ordre de 103°C pendant une durée nécessaire pour obtenir une masse sensiblement constante de ladite matière ligno-cellulosique.

Avantageusement et selon l'invention, on réalise la dispersion aqueuse par addition volontaire d'enzyme(s) de dégradation à la matière ligno-cellulosique solide. Dans un procédé selon l'invention, il est possible de préparer préalablement une solution aqueuse de l'enzyme de dégradation, puis d'ajouter ladite solution aqueuse d'enzyme de dégradation à la matière ligno-cellulosique solide. Dans une deuxième variante d'un procédé selon l'invention, on prépare d'abord la dispersion aqueuse de la matière ligno-cellulosique solide, puis on ajoute à ladite dispersion aqueuse une quantité d'enzyme de dégradation.

Avantageusement et selon l'invention, la matière ligno-cellulosique solide comprend :
- une proportion massique de cellulose, exprimée en poids sec de cellulose et en poids sec de ladite matière ligno-cellulosique solide, comprise entre 20 % et 99 % -notamment comprise entre 20 % et 98 %, de préférence comprise entre 20 % et 90 %, en particulier comprise entre 30 % et 60 %-;
- une proportion massique d'hémicelluloses, exprimée en poids sec d'hémicelluloses et en poids sec de la matière ligno-cellulosique solide, comprise entre 15 % et 50 % ;
- une proportion massique de lignines, exprimée en poids sec de lignines et en poids sec de la matière ligno-cellulosique, comprise entre 0,1 % et 30 %.

Avantageusement et selon l'invention, la dispersion aqueuse comprend une proportion massique d'enzyme(s) de dégradation par rapport à la matière sèche de la matière ligno-cellulosique solide comprise entre 1 % et 20 %.

Avantageusement et selon l'invention, on réalise le traitement mécano-enzymatique à une température comprise entre 20°C et 80°C -notamment comprise entre 30°C et 70°C, de préférence comprise entre 45°C et 55°C-.

Avantageusement et selon l'invention, le réacteur de malaxage est un extrudeur bi-vis.

Dans toute la suite, l'expression "extrudeur bi-vis" désigne un dispositif de malaxage d'une matière -notamment d'une matière ligno-cellulosique solide-, ledit dispositif de malaxage comprenant deux vis co-pénétrantes à pas direct ou à pas inversé entrainées en rotation en synchronisme à l'intérieur d'une enceinte -notamment d'une enceinte tubulaire- et présentant en section droite transversale une forme bilobée. Avantageusement, l'enceinte et les vis co-pénétrantes sont formées en acier nitruré ou en alliage industriel adapté aux conditions opératoires en particulier d'abrasivité et de corrosion. Chacune des vis co-pénétrantes est formée de tronçons de vis s'étendant axialement et successivement sur un axe cannelé et ménageant axialement des zones successives de traitement de la matière ligno-cellulosique solide entre une zone de chargement de l'extrudeur bi-vis et une zone de sortie.

Selon un mode préférentiel de réalisation d'un procédé selon l'invention, on utilise un extrudeur bi-vis à titre de réacteur de malaxage. Un avantage de l'utilisation d'un extrudeur bi-vis comme réacteur de malaxage vient de la rapidité de traitement. En effet, selon les conditions de mise en œuvre de l'invention, quelques minutes à quelques dizaines de minutes de traitement mécano-enzymatique d'une matière ligno-cellulosique dans un extrudeur bi-vis suffisent pour permettre une hydrolyse au moins partielle de la matière ligno-cellulosique solide.

Le profil de vis -défini par l'enchainement, la forme et le pas des éléments constitutifs de la bi-vis de l'extrudeur bi-vis- et la vitesse de rotation de la bi-vis sont choisis pour obtenir un effet de malaxage et/ou de broyage, et/ou de cisaillement de la dispersion aqueuse de la matière ligno-cellulosique et pour ménager un temps de séjour de ladite dispersion aqueuse dans l'extrudeur bi-vis, qui sont adaptés pour permettre une hydrolyse au moins partielle de la matière ligno-cellulosique solide.

Dans un procédé selon l'invention, la matière ligno-cellulosique solide subit une déstructuration partielle lors du traitement mécano-enzymatique de malaxage et/ou de broyage et/ou de cisaillement de ladite matière cellulosique solide mais aussi une attaque enzymatique simultanée de la matière cellulosique solide à une température comprise entre 20°C et 80°C.

L'effet de malaxage et/ou de broyage et/ou de cisaillement sont ici obtenus grâce à un déplacement entretenu d'au moins un organe mécanique rigide au sein et au contact de ladite matière cellulosique solide. Ce déplacement est dit entretenu du fait qu'il perdure pendant ledit traitement mécano-enzymatique : il peut être continu ou discontinu, il peut se répéter de manière régulière ou irrégulière.

Pour la mise en œuvre d'un traitement mécano-enzymatique selon l'invention, un tel extrudeur bi-vis présente l'intérêt de permettre une automatisation de l'ensemble des étapes et la réalisation de ce traitement en une opération unique et continue.

L'emploi d'un extrudeur bi-vis permet avantageusement un contrôle précis d'un grand nombre de paramètres de fonctionnement d'un procédé selon l'invention (température de traitement, mode et force de compression, de détente et de cisaillement, temps de traitement...). En effet, en modifiant certaines caractéristiques structurelles et/ou certaines caractéristiques de fonctionnement de l'extrudeur bi-vis, l'opérateur intervient sur les paramètres du procédé.

A titre d'exemple de caractéristiques structurelles d'un extrudeur bi-vis au niveau desquelles un opérateur peut intervenir, et qui sont généralement déterminées et fixées avant sa mise en marche (et qui ne sont pas normalement modifiables pendant son fonctionnement), on cite en particulier le profil des vis, qui dépend essentiellement de la forme du filet des vis (qui peut être par exemple trapézoïdal, conjugué, simple ou double...) et du pas de vis. Chacune de ces vis peut également présenter différents tronçons (ou segments) qui peuvent éventuellement différer les uns des autres, par la forme du filet et/ou par le pas de vis. Eventuellement, certains de ces tronçons constitutifs de ces vis peuvent également correspondre à des éléments malaxeurs monolobes ou bilobes.

Parmi ces caractéristiques de fonctionnement d'un extrudeur bi-vis mis en œuvre dans un procédé selon l'invention au niveau desquelles un opérateur peut intervenir à tout moment (aussi bien lorsque cet équipement est à l'arrêt que lorsqu'il est en marche), on peut citer :
- la vitesse de rotation des vis ;
- la température de l'enceinte (ou fourreau) ; des températures particulières peuvent être fixées dans différents modules s'étendant longitudinalement dans cette enceinte.

Le grand choix dans les caractéristiques structurelles et de fonctionnement d'un tel extrudeur bi-vis permet une grande liberté d'ajustement des conditions de mise en œuvre d'un procédé selon l'invention, et en particulier de définir des conditions optimales (par exemple la température, la force de cisaillement, la durée du traitement) spécifiques à chacune des matières ligno-cellulosiques solides de départ choisie. Avantageusement, la bi-vis d'un extrudeur bi-vis utilisée dans le cadre de l'invention, peut comprendre au moins deux tronçons qui diffèrent par leur profil de vis.

Ces caractéristiques structurelles des vis de l'extrudeur bi-vis sont adaptées pour permettre non seulement un acheminement de la matière cellulosique solide de la dispersion aqueuse le long du corps de l'extrudeur bi-vis, mais aussi de former, lors de cet acheminement de la matière cellulosique, des zones de compression et/ou de malaxage et/ou de broyage et/ou de cisaillement et/ou de gonflement par hydratation de la matière ligno-cellulosique solide de la dispersion aqueuse et une attaque enzymatique de la matière ligno-cellulosique solide de la dispersion aqueuse.

En effet, contrairement aux préjugés connus dans l'état de la technique, les inventeurs ont observé qu'en dépit des conditions de malaxage et de broyage -notamment de compression/détente successives et de cisaillement-rencontrées dans l'extrudeur bi-vis, et de la concentration élevée en substrat (matière cellulosique solide) dans l'extrudeur bi-vis, les enzymes utilisées dans un traitement mécano-enzymatique conforme à l'invention présentent une activité enzymatique préservée et en tout cas suffisante pour former rapidement des saccharides solubilisés, lors de la réalisation du traitement mécano-enzymatique en extrudeur bi-vis.

Avantageusement et selon l'invention, on choisit l'(les) enzyme(s) de dégradation de la matière ligno-cellulosique solide dans le groupe formé des cellulases, -notamment des endoglucanases, des exoglucanases et des β-glucosidases-.

Avantageusement, on choisit la cellulase dans le groupe formé des enzymes de la classe E.C.3.2.1. -notamment de la classe E.C.3.2.1.4., de la classe E.C.3.2.1.6., de la classe E.C.3.2.1.21., de la classe E.C.3.2.1.92., de la classe E.C.3.2.1.176.- de la classification des enzymes. Avantageusement, la dispersion aqueuse de la matière ligno-cellulosique solide comprend au moins une enzyme lytique adaptée pour permettre une rupture d'au moins une liaison covalente β-1,4 entre deux unités D-glucoside de la cellulose de la matière ligno-cellulosique solide et sa transformation en saccharides hydrosolubles.

Avantageusement et selon l'invention, on choisit au moins une enzyme de dégradation de la matière ligno-cellulosique solide dans le groupe formé des enzymes de dégradation des lignines.

Avantageusement, on choisit l'enzyme de dégradation de la lignine dans le groupe formé des laccases -notamment de la classe E.C.1.10.3.2. de la classification des enzymes-, des peroxydases -notamment de la classe E.C.1.11.1.7. et de la classe E.C.1.11.1.9. de la classification des enzymes-.

Avantageusement et selon l'invention, qu'on choisit au moins une enzyme de dégradation de la matière ligno-cellulosique solide dans le groupe formé des enzymes de dégradation des hémicelluloses.

Avantageusement, on choisit l'enzyme de dégradation des hémicelluloses dans le groupe formé des enzymes de la classe E.C.3.2.1. -notamment de la classe E.C.3.2.1.8., de la classe E.C.3.2.1.37. et de la classe E.C.3.2.1.89.- de la classification des enzymes.

Avantageusement et selon l'invention, lors du traitement mécano-enzymatique, on maintient le pH de la dispersion aqueuse à une valeur comprise entre pH 4 et pH 7 -notamment entre pH 5,0 et pH 6,0-.

Avantageusement et selon l'invention, on choisit la matière ligno-cellulosique solide dans le groupe formé de tout ou partie d'un plant de maïs -notamment dans le groupe formé des spathes, des rafles et des tiges-, d'une paille de céréale -notamment d'orge-, d'un déchet de fabrication de téquila -en particulier d'*Agave tequilana*-, de bagasse d'agave pour la production d'inuline, de bagasse de canne à sucre, d'un résidu de production d'huile de palme et d'un tourteau d'une plante oléagineuse.

Avantageusement et selon l'invention, on soumet la solution d'hydrolyse contenant les saccharides -notamment des monosaccharides et des oligosaccharides- solubilisés à une étape de fermentation enzymatique des saccharides solubilisés. Avantageusement, on réalise une étape de fermentation alcoolique des saccharides et du D-glucose de la solution d'hydrolyse et sa transformation en éthanol.

Avantageusement et selon l'invention, préalablement au traitement mécano-enzymatique, on ajoute à la composition intermédiaire un volume d'une solution, dite solution acide, aqueuse d'au moins un acide minéral apte à diminuer le pH de la composition intermédiaire et à former une composition intermédiaire neutralisée apte à être soumise au traitement mécano-enzymatique.

Avantageusement, le pH de la composition intermédiaire neutralisée est compris entre pH 6 et pH 7.

Avantageusement et selon l'invention, on réalise le traitement mécano-chimique et le traitement mécano-enzymatique successivement dans le même réacteur de malaxage -notamment le même extrudeur bi-vis-. Pour ce faire, l'extrudeur bi-vis présente :
- une zone amont de traitement mécano-chimique de la matière ligno-cellulosique solide, et ;
- une zone intermédiaire de lavage et de séparation liquide/solide d'une solution aqueuse riche en sels issus de la neutralisation et en hémicelluloses, et ;
- une zone aval de traitement mécano-enzymatique.

Avantageusement, on réalise le traitement mécano-chimique et le traitement mécano-enzymatique successivement dans des réacteurs de malaxage différents. Dans un mode particulier de réalisation de l'invention, il est possible d'utiliser un seul et même extrudeur pour réaliser lors d'un premier passage le traitement mécano-chimique d'une matière ligno-cellulosique solide et lors d'un deuxième passage le traitement mécano-enzymatique de la matière ligno-cellulosique solide.

Avantageusement et selon l'invention, la composition intermédiaire neutralisée présente une proportion massique de matière sèche comprise entre 10 % et 40 %.

Avantageusement et selon l'invention, on soumet la composition intermédiaire neutralisée au traitement mécano-enzymatique sans procéder à aucune étape préalable de fractionnement -notamment de séparation liquide/solide- de la matière ligno-cellulosique hydratée et d'une phase aqueuse.

Avantageusement et selon l'invention, on réalise une étape de séparation liquide/solide d'un résidu solide de la matière ligno-cellulosique solide et de la solution d'hydrolyse contenant des saccharides hydrosolubles.

Avantageusement et selon l'invention, on réalise une étape de séparation liquide/solide d'un résidu solide de la matière ligno-cellulosique solide et de la solution d'hydrolyse contenant des composés phénoliques issus des lignines de la matière ligno-cellulosique solide.

Dans un mode particulier de réalisation de l'invention, on soumet la solution d'hydrolyse contenant les saccharides hydrosolubles à une étape de fermentation -notamment une fermentation alcoolique- des saccharides hydrosolubles en vue de la formation d'éthanol.

L'invention s'étend par ailleurs à une solution aqueuse de saccharides hydrosolubles et de(s) l'enzyme(s) de dégradation de la matière ligno-cellulosique solide obtenue par un procédé selon l'invention et adaptée pour pouvoir être recyclée dans un procédé de traitement mécano-enzymatique ultérieur d'une matière ligno-cellulosique solide.

L'invention vise en outre une solution aqueuse de saccharides susceptible d'être obtenue par un procédé selon l'invention. L'invention vise aussi une solution aqueuse de saccharides hydrosolubles obtenue directement par un procédé selon l'invention.

L'invention concerne également un procédé de fabrication d'une composition de saccharides hydrosolubles et une solution aqueuse de saccharides caractérisés en combinaison par tout ou partie des caractéristiques mentionnées ci-dessus ou ci-après.

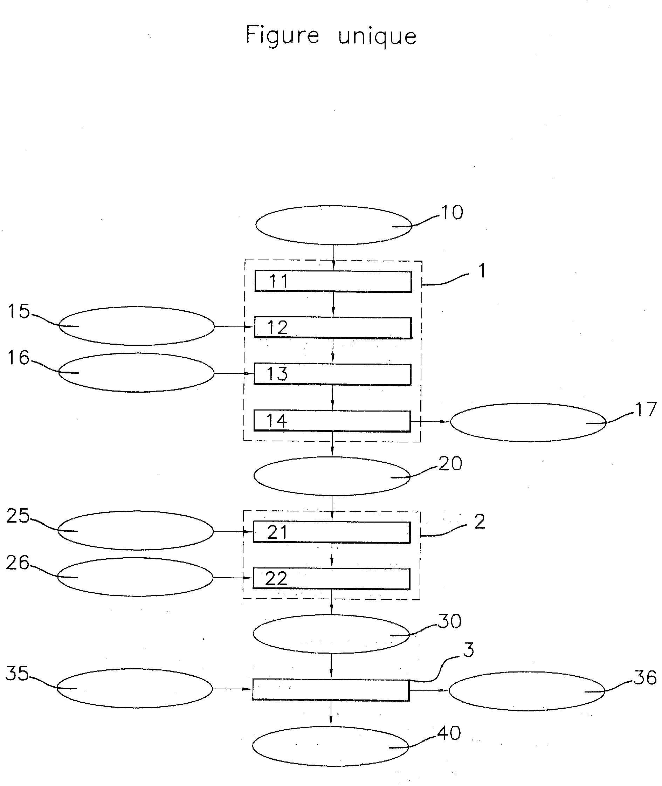

D'autres buts, caractéristiques et avantages de l'invention apparaîtront à la lecture de la description suivante qui se réfère à la figure 1 annexée illustrant un mode de réalisation d'un procédé selon l'invention, et des exemples ci-après donnés uniquement à titre indicatif d'un mode de mise en œuvre d'un procédé selon l'invention.

Dans un procédé de fabrication d'une composition de saccharides hydrosolubles selon l'invention, représenté en figure 1, on choisit une matière 10 ligno-cellulosique solide, en particulier une matière 10 ligno-cellulosique solide issue de la biomasse. Une telle matière 10 ligno-cellulosique solide présente avantageusement un taux de matière sèche compris entre 15 % et 99 %.

On introduit en continu ladite matière 10 ligno-cellulosique solide dans un premier dispositif de malaxage formé d'un extrudeur bi-vis adapté pour pouvoir soumettre la matière 10 ligno-cellulosique solide successivement aux étapes 11, 12, 13, 14 d'un traitement 1 mécano-chimique. On introduit la matière 10 ligno-cellulosique solide dans le premier extrudeur avec un débit adapté à la vitesse de rotation des vis du premier extrudeur bi-vis et à la vitesse souhaitée d'acheminement de la matière 10 ligno-cellulosique solide dans le premier extrudeur bi-vis.

Le premier extrudeur bi-vis est adapté pour permettre un ajustement de la température de la matière 10 ligno-cellulosique solide à chacune des étapes 11, 12, 13, 14 du traitement 1 mécano-chimique. L'étape 11 du traitement 1 mécano-chimique est une étape de traitement essentiellement mécanique dans lequel la matière 10 ligno-cellulosique solide subit dans l'extrudeur bi-vis des étapes successives de compression, de détente et de cisaillement. Lors de cette étape 11 initiale du traitement 1 mécano-chimique, on chauffe la matière 10 ligno-cellulosique solide de façon à atteindre une température de l'ordre de 100°C. Lors de l'étape 12 du traitement 1 mécano-chimique, on ajoute à la matière 10 ligno-cellulosique solide, sous traitement mécanique et à une température de l'ordre de 100°C, une solution 15 alcaline -notamment d'hydroxyde de sodium-. L'étape 12 d'addition de la solution 15 alcaline est adaptée pour permettre le mélange de ladite solution 15 alcaline et de la matière 10 ligno-cellulosique solide et, le cas échéant, une hydrolyse partielle des lignines de la matière 10 ligno-cellulosique solide.

On forme, à l'issue de cette étape 12 d'hydrolyse alcaline, une composition intermédiaire comprenant une matière ligno-cellulosique, dite matière ligno-cellulosique hydratée, solide et de digestibilité enzymatique -notamment de digestibilité enzymatique de la cellulose- qui est augmentée par rapport à la digestibilité enzymatique -notamment par rapport à la digestibilité enzymatique de la cellulose- de la matière 10 ligno-cellulosique solide.

Lors de l'étape 13 subséquente du traitement 1 mécano-chimique, on ajoute en continu à la composition intermédiaire une quantité d'une solution 16 acide adaptée pour neutraliser ladite composition intermédiaire et pour ajuster le pH de la composition intermédiaire à une valeur de l'ordre de pH 6 à pH 7. On réalise une telle neutralisation avec une solution aqueuse d'un acide minéral, en particulier avec une solution aqueuse d'acide phosphorique de façon à former une composition intermédiaire neutralisée.

Dans le procédé selon l'invention représenté en figure 1, on réalise ensuite une étape 14 de séparation solide/liquide d'un filtrat 17 riche en composés phénoliques et en hémicelluloses de la matière 10 ligno-cellulosique solide et d'un extrudat 20 solide enrichi en fibres cellulosiques hydratées présentant un taux de cellulose cristalline de type II augmenté de 45% à 60% par rapport à la matière 10 ligno-cellulosique solide de départ. Une telle étape 14 de séparation solide/liquide est cependant facultative.

Dans un traitement 2 mécano-enzymatique subséquent d'un extrudat 20 solide enrichi en fibres cellulosiques hydratée, on introduit ledit extrudat 20 solide dans un second extrudeur bi-vis ou dans un tronçon supplémentaire du même extrudeur. Le second extrudeur bi-vis peut être identique ou similaire au premier extrudeur bi-vis. En tout état de cause, le second extrudeur-bi-vis est adapté pour permettre un traitement mécanique de l'extrudat 20 solide par compression/détente successives et par cisaillement. On introduit l'extrudat 20 solide dans le second extrudeur bi-vis avec un débit adapté à la vitesse de rotation des vis de l'extrudeur bi-vis et à la vitesse souhaitée d'acheminement de l'extrudat 20 solide dans le second extrudeur bi-vis.

Lors du traitement 2 mécano-enzymatique, on réalise une première étape 21 d'introduction d'une solution 25 aqueuse d'au moins une enzyme d'hydrolyse d'au moins un constituant de la matière 10 ligno-cellulosique solide -notamment de la cellulose- dans le second extrudeur bi-vis. On réalise un telle introduction 21 de la solution 25 enzymatique dans le second extrudeur bi-vis avec un débit adapté à la vitesse de rotation des vis du second extrudeur bi-vis et à la vitesse souhaitée d'acheminement de la dispersion de l'extrudat 20 solide dans la solution 25 enzymatique.

En outre, on ajuste la température de la dispersion de l'extrudat 20 solide dans la solution 25 enzymatique à une valeur adaptée pour permettre une activité enzymatique optimale. En tout état de cause, dans un procédé selon l'invention, on introduit la solution 25 enzymatique dans le second extrudeur bi-vis contenant un extrudat 20 solide concentré. Selon le procédé représenté en figure 1, il est possible de procéder à une étape supplémentaire 22 d'introduction d'une solution 26 aqueuse d'au moins une enzyme d'hydrolyse de la cellulose. On réalise une telle introduction de la solution 26 enzymatique avec un débit adapté à la vitesse de rotation des vis du second extrudeur bi-vis et à la vitesse souhaitée d'acheminement de la dispersion de l'extrudat 20 solide dans la solution enzymatique. Il est possible que les solutions 25, 26 enzymatiques comprennent une même composition enzymatique ou des compositions enzymatiques distinctes. Il est possible de réaliser les étapes successives d'hydrolyse à la même température ou à des températures distinctes, chaque température étant choisie selon la température optimale d'hydrolyse de chaque composition enzymatique. A l'issue de ce traitement 2 mécano-enzymatique, on forme un hydrolysat 30 comprenant une fraction 40 solide comprenant des fibres de cellulose insolubles, des fibres de cellulose hydrosolubles, des saccharides, des enzymes des solutions 25, 26 enzymatiques et le cas échéant des composés phénoliques de dégradation des lignines et des produits de dégradation des hémicelluloses de la matière 10 ligno-cellulosique solide.

Dans le procédé selon l'invention représenté en figure 1, on soumet l'hydrolysat 30 à une étape 3 de séparation liquide/solide et de lavage de ladite fraction 40 solide et d'une solution 35 aqueuse. On réalise en particulier cette étape 3 de séparation liquide/solide et de lavage dans un troisième extrudeur bi-vis ou dans un tronçon supplémentaire du même extrudeur bi-vis comprenant un module de filtration adapté pour permettre une séparation de la fraction 40 solide de l'hydrolysat 30 et d'un filtrat 36 liquide aqueux comprenant des fibres de cellulose hydrosolubles, des saccharides, au moins une partie des enzymes des solutions 25, 26 enzymatiques, et le cas échéant, des composés phénoliques issus de la dégradation des lignines et des hémicelluloses et des produits de dégradation des hémicelluloses. Une telle étape de séparation liquide/solide est néanmoins facultative. En outre, avantageusement, il est possible d'utiliser un extrudeur bi-vis unique présentant une succession de tronçons, chaque tronçon étant adapté pour permettre un traitement en continu de la matière ligno-cellulosique solide.

Dans une première variante non représentée d'un procédé selon l'invention, on réalise un traitement de réduction de la granulométrie de la matière ligno-cellulosique solide préalablement au traitement mécano-chimique. On réalise cette étape de diminution de la granulométrie de la matière ligno-cellulosique solide par tout traitement connu de l'homme du métier par exemple dans un broyeur, un hachoir ou tout autre dispositif de réduction de la granulométrie.

Dans une deuxième variante non représentée d'un procédé selon l'invention, on collecte le filtrat 36 liquide aqueux obtenu lors de l'étape de séparation liquide/solide, ledit filtrat 36 liquide aqueux comprenant des fibres de cellulose hydrosolubles, des saccharides, des enzymes des solutions enzymatiques et le cas échéant des composés phénoliques issus de la dégradation des lignines et des produits de dégradation des hémicelluloses et on recycle dans l'extrudeur bi-vis ce filtrat liquide aqueux contenant des enzymes de la(des) solution(s) enzymatiques lors d'une étape subséquente de traitement mécano-enzymatique selon l'invention. On peut également faire subir à la fraction 40 un second cycle de traitement reprenant l'intégralité des étapes précédemment décrites.

Dans une troisième variante non représentée d'un procédé selon l'invention, on réalise un traitement mécano-enzymatique d'une matière ligno-cellulosique solide en introduisant ladite matière ligno-cellulosique directement dans un extrudeur bi-vis. Lors du traitement mécano-enzymatique de ladite matière ligno-cellulosique solide, on réalise une première étape d'introduction d'une solution aqueuse d'au moins une enzyme d'hydrolyse d'au moins un constituant de la matière ligno-cellulosique solide -notamment de la cellulose-. On réalise une telle introduction de la solution enzymatique dans l'extrudeur bi-vis avec un débit adapté à la vitesse de rotation des vis de l'extrudeur bi-vis et à la vitesse souhaitée d'acheminement de la dispersion de la matière ligno-cellulosique solide dans la solution enzymatique.

### EXEMPLE 1 : Traitement d'une matière ligno-cellulosique formée des co-produits industriels de maïs doux.

On collecte une matière ligno-cellulosique formée des parties non valorisées de plants de maïs doux, notamment des rafles égrainées et des spathes obtenues après la récolte des épis de maïs. Dans cet exemple, le coproduit de maïs doux a subi préalablement un traitement thermique de déshydratation de façon à présenter un taux de matière sèche de 92% et permettant sa conservation. On réalise une étape de broyage de ce coproduit de maïs doux dans un broyeur à marteaux VS 1 (ELECTRA, Poudenas, France) équipé d'une grille de sélection de 2 mm. Les caractéristiques du coproduit de maïs doux (spathes et épis découpés) déshydraté sont décrites ci-après :
1 Kg de coproduit de maïs doux (matière sèche) comprend :
- 0,96 Kg de matière organique ;
- 0,39 Kg de cellulose ;
- 0,36 Kg d'hémicelluloses ;
- 0,04 Kg de lignines et ;
- 0,06 Kg de matière organique hydrosoluble à chaud.

On introduit en continu le coproduit de maïs doux broyé dans un extrudeur bi-vis CLEXTRAL BC 45 avec un débit d'introduction de matière sèche dudit coproduit de maïs doux broyé dans l'extrudeur de 11,4 kg/h.

### 1) Traitement mécano-chimique

On réalise un premier traitement mécano-chimique du coproduit de maïs doux broyé dans un extrudeur bi-vis CLEXTRAL BC 45 (CLEXTRAL SA, Firminy, France) dans lequel deux vis parallèles et identiques, de profondeur de filet constante, tournent en même temps et à la même vitesse dans l'alésage d'un fourreau bilobé fixe. Dans un tel extrudeur bi-vis, les deux vis parallèles sont du type "co-pénétrantes" et adaptées pour soumettre le coproduit de maïs doux broyé à un travail mécanique de cisaillement et de mélange au moyen de séquences successives de compression, de détente et de cisaillement.

La configuration de l'extrudeur bi-vis CLEXTRAL BC 45 est décrite à titre d'exemple non limitatif dans le tableau 1 ci-après.

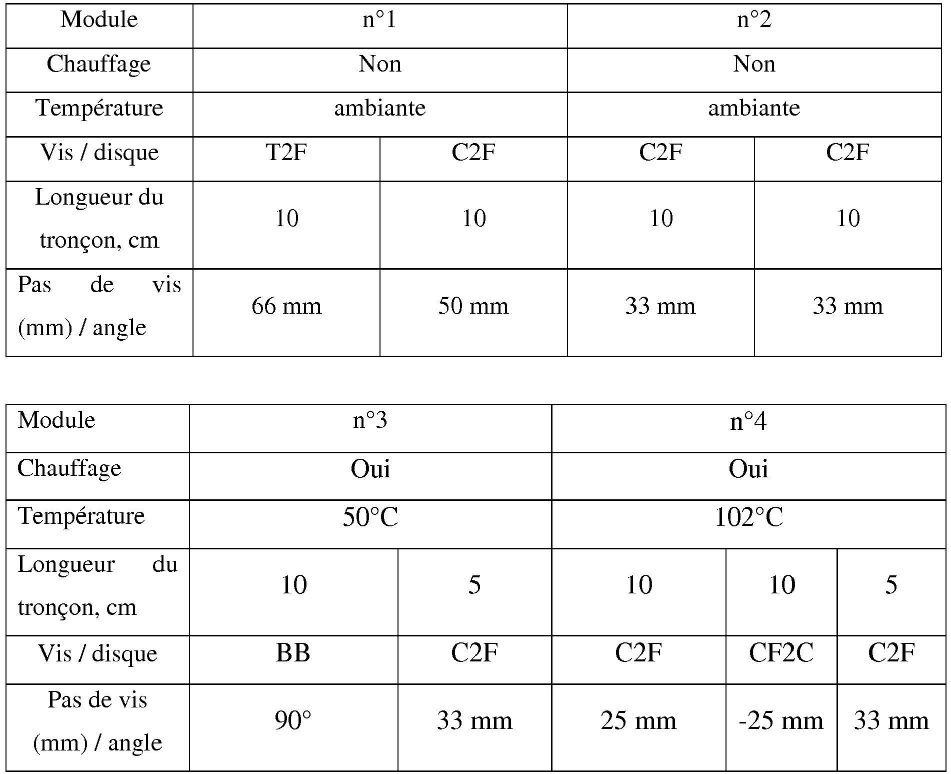

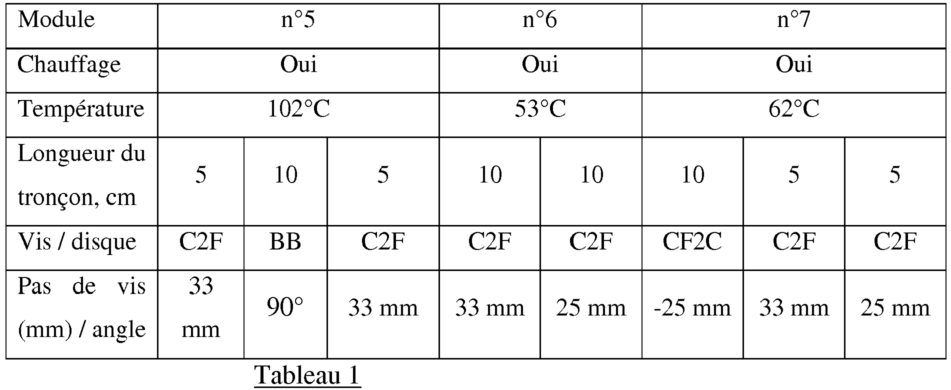

Dans la configuration d'un dispositif de traitement décrit au tableau 1 et mis en œuvre dans un procédé selon l'invention, l'extrudeur bi-vis CLEXTRAL BC 45 comprend sept modules enchainés linéairement les uns aux autres successivement. Il comprend :
- un premier module (module n°1) s'étendant en regard de la zone de chargement et comprenant :
   ∘ un tronçon de vis trapézoïdale et à double filet du type T2F et présentant un pas de vis de 66 mm adapté pour permettre l'introduction du coproduit de maïs doux et son convoyage, et ;
   ∘ un tronçon de vis conjuguée et à double filet du type C2F et présentant un pas de vis de 50 mm adapté pour permettre le convoyage du coproduit de maïs doux ;
- un deuxième module (module n°2) s'étendant en aval du module n°1 et comprenant :
   ∘ un tronçon de vis conjuguée et à double filet du type C2F et présentant un pas de vis de 33 mm adapté pour permettre le convoyage et la compression du coproduit de maïs doux;
   ∘ un deuxième tronçon de vis conjuguée et à double filet du type C2F et présentant un pas de vis de 33 mm adapté pour permettre le convoyage et la compression du coproduit de maïs doux et une entrée de composition liquide ;
- un troisième module (module n°3) thermo-régulé à 50°C et s'étendant en aval du deuxième module et comprenant :
   ∘ une série de 10 disques malaxeurs bilobés du type BB (MAL 2) montés perpendiculairement à l'axe cannelé et décalés d'un angle de 90° les uns par rapport aux autres, adapté pour permettre un mélange du coproduit de maïs doux et pour augmenter son temps de séjour dans l'extrudeur ;
   ∘ un tronçon de vis conjuguée et à double filet du type C2F et présentant un pas de vis de 33 mm adapté pour permettre le convoyage du coproduit de maïs doux ;
- un quatrième module (module n°4) thermo-régulés à 102°C, s'étendant en aval du troisième module et comprenant :
   ∘ un tronçon de vis conjuguée et à double filet du type C2F et présentant un pas de vis de 25 mm adapté pour permettre le convoyage du coproduit de maïs doux ;
   ∘ un tronçon de vis inversée du type CF2C à pas de vis de -25 mm ;
   ∘ un tronçon de vis conjuguée et à double filet du type C2F et présentant un pas de vis de 33 mm adapté pour permettre le convoyage du coproduit de maïs doux, et ;

- un cinquième module (module n°5) thermo-régulé à 102°C, s'étendant en aval du quatrième module et comprenant :
   ∘ une entrée de composition liquide, et ;
   ∘ un premier tronçon de vis conjuguée et à double filet du type C2F et présentant un pas de vis de 33 mm adapté pour permettre le convoyage du coproduit de maïs doux ;
   ∘ une série de 5 disques malaxeurs bi-lobe du type BB (MAL 2) montés perpendiculairement à l'axe cannelé et décalés d'un angle de 90° les uns par rapport aux autres, adapté pour permettre un mélange du coproduit de maïs doux et pour augmenter son temps de séjour dans l'extrudeur ;
   ∘ un deuxième tronçon de vis conjuguée et à double filet du type C2F et présentant un pas de vis de 33 mm adapté pour permettre le convoyage du coproduit de maïs doux ;
- un sixième module (module n°6) équipé d'une grille de filtration, s'étendant en aval du cinquième module et comprenant :
   ∘ un tronçon de vis conjuguée et à double filet du type C2F et présentant un pas de vis de 33 mm,
   ∘ une sortie de composition liquide (filtrat) s'étendant en regard de ce tronçon C2F ;
   ∘ un tronçon de vis conjuguée et à double filet du type C2F et présentant un pas de vis de 25 mm ;
- un septième module (module n°7) de sortie thermo-régulé à 62°C, s'étendant en aval du sixième module et comprenant :
   ∘ un tronçon de vis inversée de type CF2C à pas de vis de -25 mm destiné à presser la matière, et ;
   ∘ un tronçon de vis conjuguée à double filet du type C2F et présentant un pas de vis de 33 mm adapté pour permettre une reprise du coproduit de maïs doux en aval du contrefilet ;
   ∘ un tronçon de vis conjuguée et à double filet du type C2F et présentant un pas de vis de 25 mm permettant l'évacuation du coproduit de maïs doux vers l'extérieur du fourreau.

Un tel profil de vis est choisi pour permettre successivement l'introduction du coproduit de maïs doux dans l'extrudeur bi-vis, puis, l'introduction de la solution de soude, son mélange avec le coproduit de maïs doux, pour ménager un temps de contact entre le coproduit de maïs doux et la soude, puis la neutralisation du milieu par incorporation d'acide, et, le cas échéant, la séparation liquide/solide par filtration d'une partie des composés solubilisés.

Dans un tel dispositif, le coproduit de maïs doux progresse axialement en passant d'une vis à l'autre et en suivant un trajet en "huit ouvert" entre la zone de chargement et la zone de sortie de la composition intermédiaire neutralisée. La vitesse de rotation des vis est adaptée pour éviter un engorgement de l'extrudeur bi-vis. Dans cet exemple, la vitesse de rotation des vis est de 110 rpm.

On introduit en continu, au niveau du quatrième module, une solution aqueuse alcaline formée de 4 g d'hydroxyde de sodium (NaOH) dans 100 g de solution et selon un débit de 24 Kg/h de ladite solution alcaline. Une telle addition de la solution aqueuse alcaline au coproduit de maïs doux permet une addition de 90 g d'hydroxyde de sodium par kilogramme de matière sèche du coproduit de maïs doux.

Le profil de l'extrudeur bi-vis est adapté pour permettre successivement l'introduction du coproduit de maïs doux dans l'extrudeur bi-vis, son malaxage mécanique, puis l'introduction de la solution alcaline, le mélange de la solution alcaline et du coproduit de maïs doux sous contrainte mécanique et l'introduction ultérieure de la solution acide de neutralisation.

On introduit en continu, au niveau du cinquième module, une solution aqueuse acide formée de 2,4 g d'acide phosphorique (H₃PO₄) dans 100 g de solution et avec un débit de 38 Kg/h de ladite solution acide. Une telle addition de la solution aqueuse acide dans l'extrudeur bi-vis permet une addition de 80 g d'acide phosphorique par kilogramme de matière sèche du coproduit de maïs doux.

On réalise une étape de séparation liquide/solide par filtration de la composition intermédiaire neutralisée apte à former un filtrat aqueux qui s'écoule par la sortie de composition liquide (filtrat) du sixième module.

En sortie du septième module de l'extrudeur bi-vis CLEXTRAL BC 45 à l'issue du traitement mécano-chimique, on collecte une composition intermédiaire neutralisée à pH 6.

1 Kg de matière sèche de cette composition intermédiaire neutralisée contient :
- 0,93 Kg de matière organique ;
- 0,45 Kg de cellulose ;
- 0,28 Kg d'hémicelluloses ;
- 0,04 Kg de lignines et ;
- 0,08 Kg de matière organique hydrosoluble à chaud.

### 2) Traitement mécano-enzymatique

On reprend cette composition intermédiaire neutralisée et on l'introduit dans un extrudeur bi-vis CLEXTRAL BC21 pour un traitement mécano-enzymatique.

La configuration de l'extrudeur bi-vis CLEXTRAL BC 21 est décrite à titre d'exemple non limitatif dans le tableau 2 ci-après.

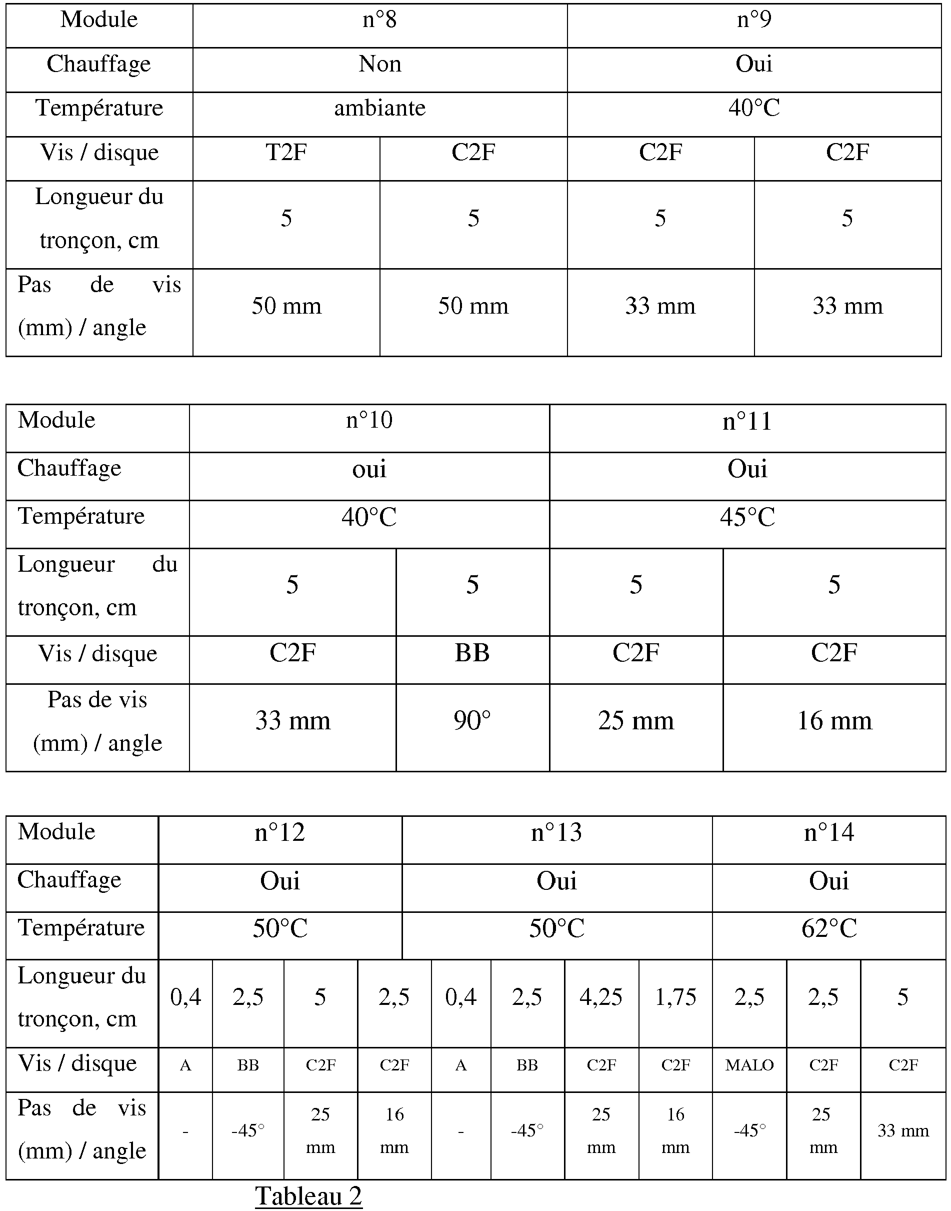

Dans la configuration d'un dispositif de traitement décrit au tableau 2 et mis en œuvre dans un procédé selon l'invention, l'extrudeur bi-vis CLEXTRAL BC 21 comprend sept modules enchainés linéairement les uns aux autres successivement. Il comprend :
- un huitième module (module n°8) s'étendant en regard de la zone de chargement et comprenant deux tronçons successifs de vis trapézoïdale et à double filet du type T2F et présentant un pas de vis de 50 mm adapté pour permettre l'introduction de la composition intermédiaire neutralisée et son convoyage, et ;
- un neuvième module (module n°9) thermo-régulé à 40°C s'étendant en aval du huitième module et comprenant deux tronçons de vis conjuguées et à double filet du type C2F et présentant un pas de vis de 33 mm adapté pour permettre le convoyage et la compression de la composition intermédiaire neutralisée et une entrée de composition enzymatique liquide ;
- un dixième module (module n°10) thermo-régulé à 40°C et s'étendant en aval du neuvième module et comprenant :
   ∘ un tronçon de vis conjuguée et à double filet du type C2F et présentant un pas de vis de 33 mm adapté pour permettre le convoyage de la composition intermédiaire neutralisée ;
   ∘ une série de 10 disques malaxeurs bilobés du type BB (MAL 2) montés perpendiculairement à l'axe cannelé et décalés d'un angle de 90° les uns par rapport aux autres, adapté pour permettre un mélange de la composition intermédiaire neutralisée et pour augmenter son temps de séjour dans l'extrudeur ;
- un onzième module (module n°11) thermo-régulés à 45°C, s'étendant en aval du dixième module et comprenant :
   ∘ un tronçon de vis conjuguée et à double filet du type C2F et présentant un pas de vis de 25 mm adapté pour permettre le convoyage de la composition intermédiaire neutralisée ;
   ∘ un tronçon de vis conjuguée et à double filet du type C2F et présentant un pas de vis de 16 mm adapté pour permettre le convoyage de la composition intermédiaire neutralisée, et ;
- un douzième module (module n°12) thermo-régulé à 50°C, s'étendant en aval du onzième module et comprenant :
   ∘ un anneau neutre, et ;
   ∘ une série de 5 disques malaxeurs bilobés du type BB (MAL 2) montés perpendiculairement à l'axe cannelé et décalés d'un angle de -45° les uns par rapport aux autres, adaptés pour permettre un mélange de la composition intermédiaire neutralisée et pour augmenter son temps de séjour dans l'extrudeur, et ;
   ∘ un tronçon de vis conjuguée et à double filet du type C2F et présentant un pas de vis de 25 mm adapté pour permettre le convoyage de la composition intermédiaire neutralisée, et ;
- un treizième module (module n°13) thermo-régulé à 50°C et s'étendant en aval du douzième module et comprenant :
   ∘ un tronçon de vis conjuguée et à double filet du type C2F et présentant un pas de vis de 16 mm,
   ∘ un anneau neutre, et ;
   ∘ une série de 5 disques malaxeurs bilobés du type BB (MAL 2) montés perpendiculairement à l'axe cannelé et décalés d'un angle de -45° les uns par rapport aux autres, et ;
   ∘ un tronçon de vis conjuguée et à double filet du type C2F et présentant un pas de vis de 25 mm,
   ∘ un tronçon de vis conjuguée et à double filet du type C2F et présentant un pas de vis de 16 mm ;
- un quatorzième module (module n°14) de sortie thermo-régulé à 50°C, s'étendant en aval du treizième module et comprenant :
   ∘ une série de disques malaxeurs mono-lobe du type MALO (MAL 2) montés perpendiculairement à l'axe cannelé et décalés d'un angle de -45° les uns par rapport aux autres, et ;
   ∘ un tronçon de vis conjuguée et à double filet du type C2F et présentant un pas de vis de 25 mm, et ;
   ∘ un tronçon de vis conjuguée et à double filet du type C2F et présentant un pas de vis de 33 mm.

On introduit la composition intermédiaire neutralisée dans l'extrudeur bi-vis CLEXTRAL BC 21 au niveau de la zone de chargement du module n°8 avec un débit de matière sèche de 1,5 Kg/h. La vitesse de rotation des vis de l'extrudeur bi-vis CLEXTRAL BC 21 est de 250 rpm.

On introduit dans l'extrudeur bi-vis CLEXTRAL BC 21 une solution d'enzymes (Sacchariseb C6, Advanced Enzyme Technologies Ltd, Californie, USA) dans un tampon aqueux de citrate à 300 mM présentant une concentration en enzyme de 3 % (v/v). Le débit d'introduction de la solution d'enzymes dans l'extrudeur bi-vis CLEXTRAL BC 21 est de 1,8 Kg de solution d'enzymes par heure. La configuration de l'extrudeur bi-vis CLEXTRAL BC 21 est adaptée pour permettre l'introduction de la composition intermédiaire neutralisée dans l'extrudeur bi-vis, le mélange intime de ladite composition intermédiaire neutralisée et de la composition enzymatique et le maintien en contact de la composition intermédiaire neutralisée et de la solution d'enzymes de façon à permettre l'hydrolyse au moins partielle de la matière organique -notamment de la cellulose- de la matière ligno-cellulosique solide.

A l'issue du traitement mécano-enzymatique, en sortie du septième module de l'extrudeur bi-vis CLEXTRAL BC21, on collecte une composition bio-extrudée (CBE).

1 Kg de matière sèche de cette composition bio-extrudée (CBE) contient :
- 0,895 Kg de matière organique, dont 22 g d'enzyme ;
- 0,38 Kg de cellulose ;
- 0,17 Kg d'hémicelluloses ;
- 0,04 Kg de lignines et ;
- 0,15 Kg de matière organique hydrosoluble à chaud.

Dans les conditions opératoires de cet exemple de traitement de coproduits de maïs doux, le traitement mécano-enzymatique permet l'hydrolyse de 10% de la cellulose de la composition intermédiaire neutralisée et 37% des hémicelluloses de la composition intermédiaire neutralisée. Il permet aussi de doubler la quantité de matière organique soluble à chaud.

En outre, la fermentation de 1Kg de composition bio-extrudée (CBE) conduit, sans ajout supplémentaire d'enzyme, à la formation de 120 g d'éthanol correspondant à :
- 56% du potentiel maximal de fermentation de la totalité des hexoses de la composition bio-extrudée (CBE), et à ;
- 49 % du potentiel maximal de fermentation des hexoses du coproduit de maïs doux de départ.

### 3) Deuxième cycle de traitements mécano-chimique et mécano-enzymatique successifs

On réalise, à l'issue de l'étape de traitement mécano-enzymatique, un traitement additionnel d'extrusion bi-vis lors duquel on introduit la composition bio-extrudée (CBE) dans un extrudeur bi-vis CLEXTRAL BC 21, sensiblement similaire à l'extrudeur bi-vis mis en œuvre pour le traitement mécano-enzymatique ci-dessus et dans lequel :
- un organe d'introduction d'eau a été aménagé dans le module 17, et ;
- une zone de filtration a été ajoutée au niveau d'un module 21 modifié par rapport au module 14 mis en œuvre lors du traitement mécano-enzymatique et dont la configuration est décrite au tableau 3 ci-après.

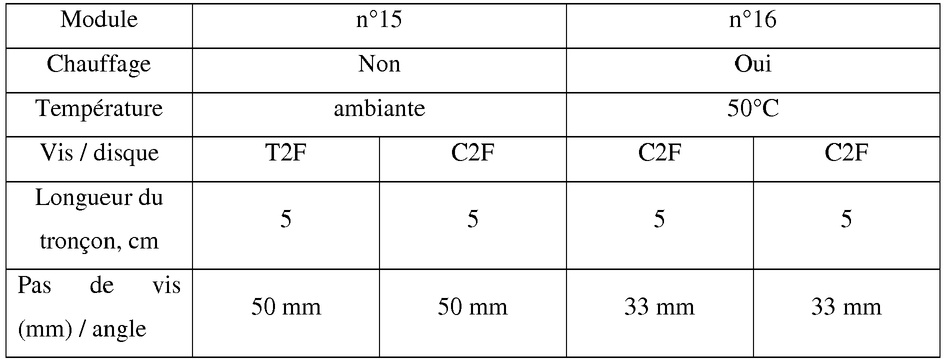

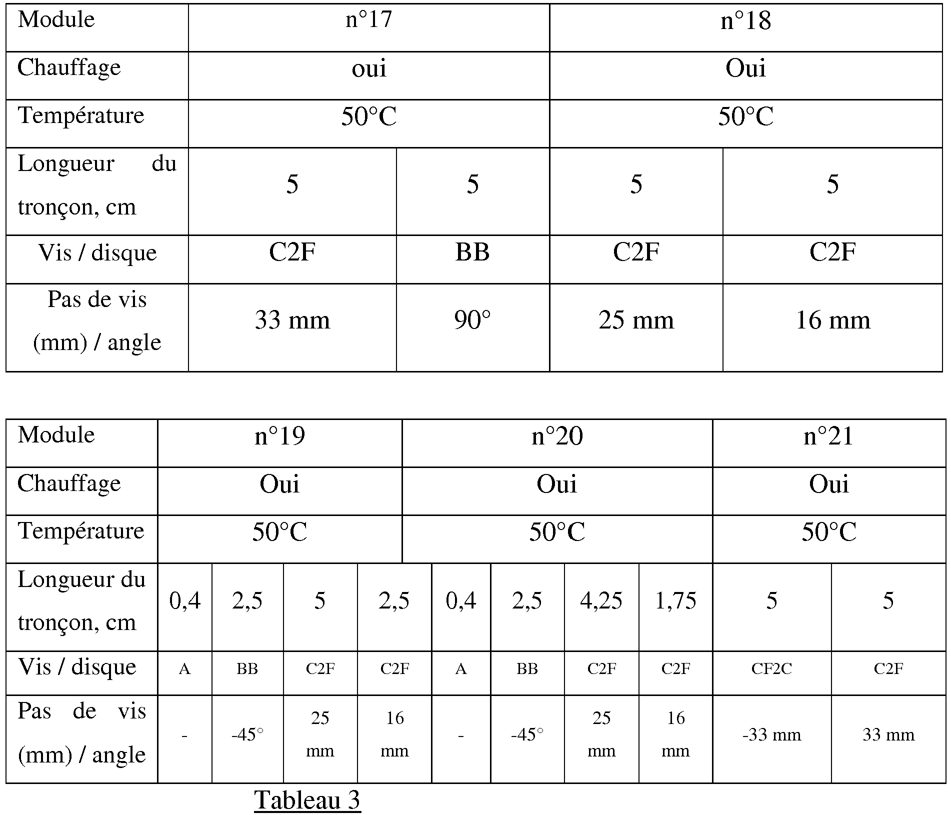

Un tel extrudeur bi-vis dédié au traitement additionnel d'extrusion bi-vis est un extrudeur bi-vis CLEXTRAL BC21 comprenant sept modules enchainés linéairement les uns aux autres successivement. L'extrudeur bi-vis CLEXTRAL BC 21 de traitement additionnel d'extrusion comprend :
- un module (module n°15) équivalent au module n°8 et ;
- un module (module n°16) équivalent au module n°9 ci-dessus, thermo-régulé à 53°C et s'étendant en aval du module n°15, et ;
- un module (module n°17) équivalent au module n°10 ci-dessus, thermo-régulé à 55°C et s'étendant en aval du module n°16 ;
- un module (module n°18) équivalent au module n°11 ci-dessus, thermo-régulés à 50°C et s'étendant en aval du module °17;
- un module (module n°19) équivalent au module n°12 ci-dessus, thermo-régulé à 53°C et s'étendant en aval du module n°18 ;
- un module (module n°20) équivalent au module n°13 ci-dessus, s'étendant en aval du module n°19 et comprenant une sortie de fluide équipée d'un organe de séparation solide/liquide par filtration présentant un diamètre de maille de 500 µm, et ;
- un module (module n°21) thermo-régulé à 56°C et s'étendant en aval du module n°20 et comprenant :
   ∘ un tronçon de vis conjuguée à double filet inversé de type CF2C et présentant un pas de vis de -33 mm, et ;
   ∘ un tronçon de vis conjuguée et à double filet du type C2F et présentant un pas de vis de 33 mm.

La vitesse de rotation des vis de l'extrudeur additionnel d'extrusion est de 250 rpm. Le débit d'introduction de la matière sèche de la composition bio-extrudée (CBE) dans l'extrudeur additionnel est de 2,7 Kg/h. Le débit d'introduction d'eau au niveau de l'entrée de composition liquide aqueuse (module n°16) est de 7Kg/h. Le rapport massique liquide/solide de la composition bio-extrudée (CBE) dans l'extrudeur additionnel est de 5,2.

En sortie du module n°21 de l'extrudeur bi-vis et à l'issue du traitement par lavage, on collecte une composition intermédiaire lavée.

1 Kg de ladite matière intermédiaire lavée contient :
- 0,95 Kg de matière organique, dont ;
- 0,49 Kg de cellulose ;
- 0,28 Kg d'hémicelluloses ;
- 0,05 Kg de lignines, et ;
- 0,02 Kg de matière organique hydrosoluble à chaud.

A l'issue de l'étape de lavage ci-dessus, on réalise un second traitement mécano-chimique de la composition intermédiaire lavée. Le traitement est sensiblement identique au traitement décrit au 1) de l'exemple 1 et adapté à l'extrudeur bi-vis CLEXTRAL BC 21 dont la configuration est décrite à titre d'exemple non limitatif dans le tableau 4 ci-après.

Les modules 24 et 26 sont équipés d'une introduction liquide destinée respectivement à l'introduction de la solution alcaline et de la solution acide. Le module 20 est un module de filtration.

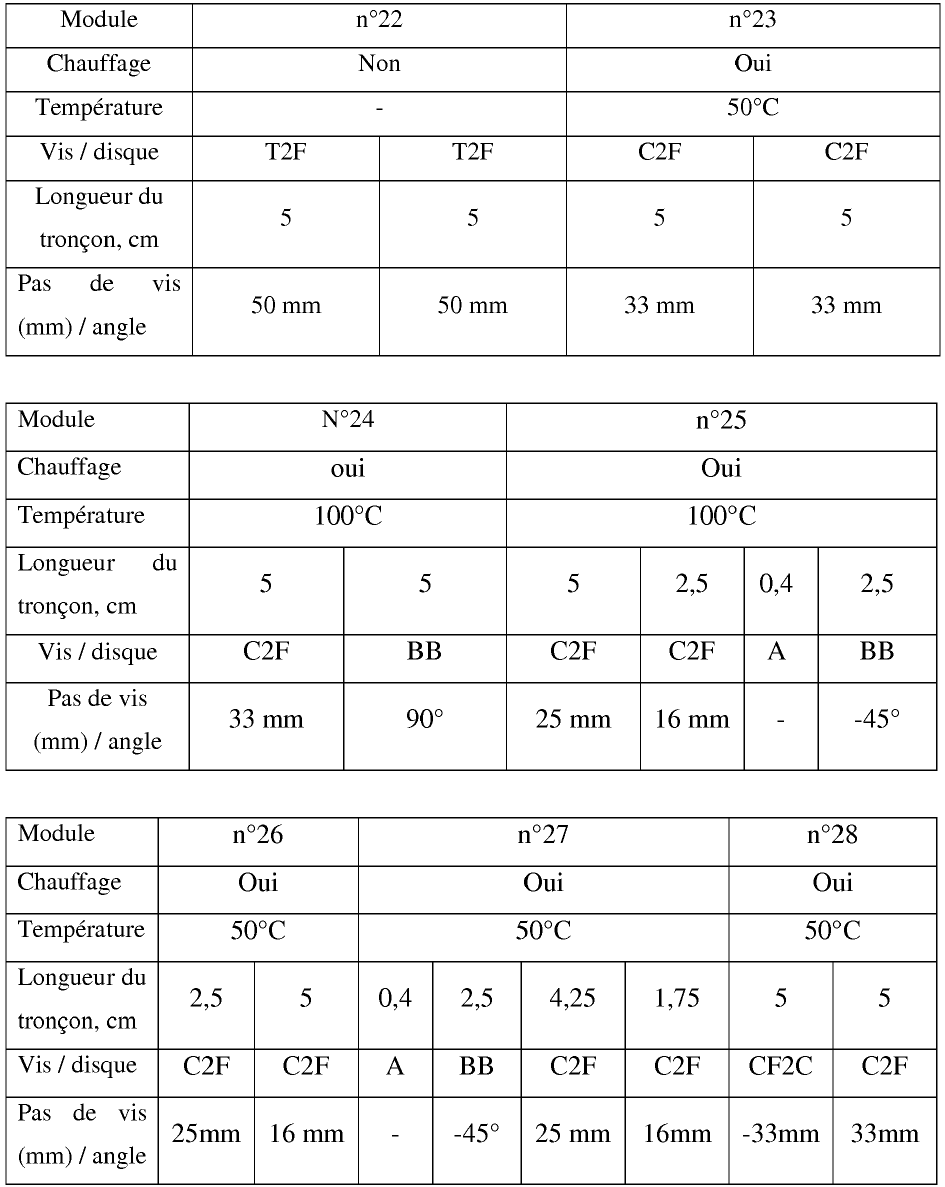

Tableau 4

Comme dans le premier traitement un tel profil de vis est choisi pour permettre successivement l'introduction de la composition intermédiaire lavée dans l'extrudeur bi-vis, puis, l'introduction de la solution de soude, son mélange avec la composition intermédiaire, un temps de contact entre la composition intermédiaire et la soude, puis la neutralisation du milieu par incorporation d'acide, et, la séparation liquide/solide par filtration d'une partie des composés solubilisés.

La vitesse de rotation des vis est fixée à 250 rpm.

On introduit en continu la composition intermédiaire lavée au niveau du premier module de l'extrudeur bi-vis CLEXTRAL BC 21 avec un débit de 0,99 Kg/h de matière sèche. On introduit en continu, au niveau du vingt-deuxième module, une solution aqueuse alcaline formée de 4g d'hydroxyde de sodium (NaOH) dans 100g de solution et selon un débit de 4 Kg/h. Une telle addition de la solution aqueuse alcaline dans la composition intermédiaire lavée permet une addition de 160 g d'hydroxyde de sodium par kilogramme de matière sèche de la composition intermédiaire lavée.

On introduit en continu, au niveau du vingt-sixième module, une solution aqueuse acide formée d'acide phosphorique (H₃PO₄) dans l'eau à 1,8 % et avec un débit de 10 Kg/h de ladite solution acide. Une telle addition de la solution aqueuse acide dans l'extrudeur bi-vis permet une addition de 180 g d'acide phosphorique par kilogramme de matière sèche de la composition intermédiaire lavée.

On réalise une étape de séparation liquide/solide par filtration de la composition intermédiaire neutralisée apte à former un filtrat aqueux qui s'écoule par la sortie de composition liquide (filtrat) du sixième module.

En sortie du vingt-huitième module de l'extrudeur bi-vis CLEXTRAL BC 21 à l'issue du traitement mécano-chimique, on collecte une deuxième composition intermédiaire neutralisée à pH 6. 1 kg de matière sèche de cette deuxième composition intermédiaire neutralisée contient 0,94kg de matière organique, dont 0,60 kg de cellulose, 0,25 kg d'hémicelluloses, 0,04kg de lignine et 0,07kg de matière organique hydrosoluble à chaud.

On reprend cette deuxième composition intermédiaire neutralisée et on l'introduit dans un extrudeur bi-vis CLEXTRAL BC 21 pour un second traitement mécano-enzymatique réalisé dans des conditions opératoires proches du traitement mécano-enzymatique en extrudeur bi-vis CLEXTRAL BC 21.

La configuration de l'extrudeur bi-vis CLEXTRAL BC 21 utilisé pour cette étape de deuxième traitement mécano-enzymatique est décrite à titre d'exemple non limitatif dans le tableau 5 ci-après.

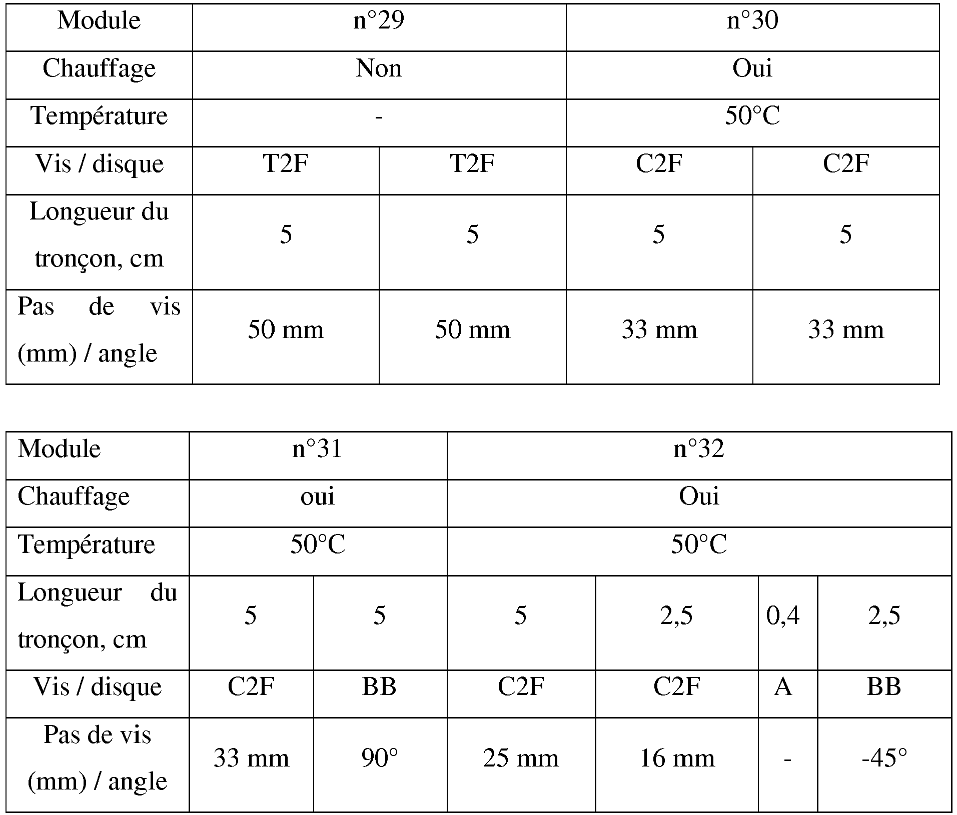

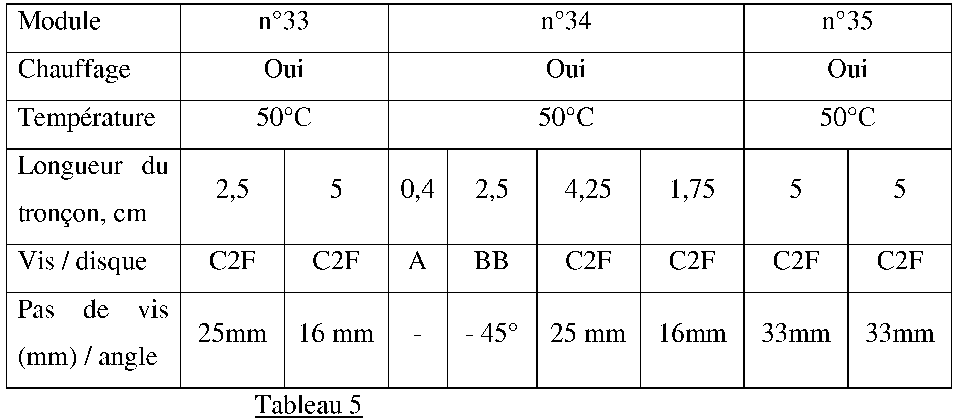

On introduit la deuxième composition intermédiaire neutralisée dans l'extrudeur bi-vis CLEXTRAL BC 21 au niveau de la zone de chargement du module n°29 avec un débit de matière sèche de 0,87 Kg/h. La vitesse de rotation des vis de l'extrudeur bi-vis CLEXTRAL BC 21 est de 250 rpm.

On introduit dans l'extrudeur bi-vis CLEXTRAL BC 21 une solution d'enzymes Sacchariseb C6 dans un tampon aqueux de citrate à 300 mM présentant une concentration en enzyme de 3 % (m/m). Le débit d'introduction de la solution d'enzymes dans l'extrudeur bi-vis CLEXTRAL BC 21 est de 2,3 Kg de solution d'enzymes par heure. A l'issue du traitement mécano-enzymatique, en sortie du trente-cinquième module de l'extrudeur bi-vis CLEXTRAL BC 21, on collecte une deuxième composition bio-extrudée. 1 kg de matière sèche de cette deuxième composition bio-extrudée contient 0,91kg de matière organique, dont 46g d'enzymes, 0,52 kg de cellulose, 0,01 kg d'hémicelluloses, 0,03kg de lignine et 0,25 kg de matière organique hydrosoluble à chaud.

Dans les conditions opératoires de cet exemple de traitement d'une composition de biomasse de coproduits industriel de maïs doux, le second traitement mécano-enzymatique permet l'hydrolyse de 94 % des hémicelluloses de la deuxième composition intermédiaire neutralisée, accompagnée d'une augmentation d'un facteur 10 de la matière organique hydrosoluble dans la deuxième composition bio-extrudée.

La fermentation de 1kg de la deuxième composition bio-extrudée (sans ajout d'enzymes supplémentaires) conduit à la formation de 185g d'éthanol correspondant à 62% du potentiel maximal de fermentation de la totalité des hexoses de la deuxième composition bio-extrudée, soit 54.3% du potentiel maximal de fermentation de la totalité des hexoses du coproduit industriel de maïs doux initiale. Il est à noter que le filtrat de lavage contient 18.5% du potentiel maximal de fermentation du coproduit industriel de maïs doux initial.

### EXEMPLE 2: Traitement d'une composition de biomasse formée des résidus de production d'huile de palme.

On collecte une composition de biomasse formée des parties non valorisées de l'extraction de l'huile de palme. On réalise une étape de broyage de résidus de palmier lors de l'extraction de l'huile de palme dans un broyeur à marteaux VS 1 (ELECTRA, Poudenas, France) équipé d'une grille de sélection de 2 mm. 1 Kg de matière sèche de ces résidus de palmier contient 0,97 Kg de matière organique, dont 0,44 Kg de cellulose, 0,24 Kg d'hémicelluloses, 0,19 Kg de lignine et 0,04 Kg de matière organique hydrosoluble à chaud.

On fait subir à ces résidus de palmier la même succession de traitement que celle appliquée aux co-produits industriels du maïs doux citée dans l'exemple 1.

### 1) Traitement mécano-chimique

Le premier traitement mécano-chimique des résidus de palmier broyés est réalisé dans un extrudeur bi-vis CLEXTRAL BC 45 (CLEXTRAL SA, Firminy, France). L'extrudeur bi-vis CLEXTRAL BC 45 comprend une ouverture d'introduction des résidus de palmier au niveau du module n°1, une entrée d'une composition liquide alcaline au niveau du module n°2, une entrée d'une composition liquide acide au niveau du module n°6 et un dispositif de filtration au niveau du module n°6.

La température de consigne des modules est donnée au tableau 6 ci-après.

**Tableau 6**

| | Module | | | | | | |
|---|---|---|---|---|---|---|---|
| | n°1 | n°2 | n°3 | n°4 | n°5 | n°6 | n°7 |
| T°C | ambiante | ambiante | 40 | 100 | 50 | 50 | 70 |

Les conditions de traitement sont décrites dans le tableau 7 ci-après.

**Tableau 7**

| Traitement | Débit d'entrée de matière sèche (Kg/h) | Vitesse de vis (rpm) | Entrée NaOH | | Entrée H₃PO₄ | | Solution enzymatique |
|---|---|---|---|---|---|---|---|
| | | | Débit (Kg/h) | Concentration (%) | Débit (Kg/h) | Concentration (%) | Débit (Kg/h) |
| Mécano-chimique | 8,5 | 200 | 27 | 4 | 63 | 1,8 | |
| Mécano-enzymatique | 1,5 | 200 | | | | | 5,6 |

2) 1 kg de matière sèche de cette composition intermédiaire neutralisée contient 0,97kg de matière organique, dont 0,53 kg de cellulose, 0,23 kg d'hémicelluloses, 0,21 kg de lignines et 0,01kg de matière organique hydrosoluble à chaud.

### 3) Traitement mécano-enzymatique

On reprend la composition intermédiaire neutralisée et on l'introduit dans un extrudeur bi-vis CLEXTRAL BC21 pour un traitement mécano-enzymatique.

La configuration de l'extrudeur bi-vis CLEXTRAL BC 21 est décrite à titre d'exemple non limitatif dans le tableau 8 ci-après.

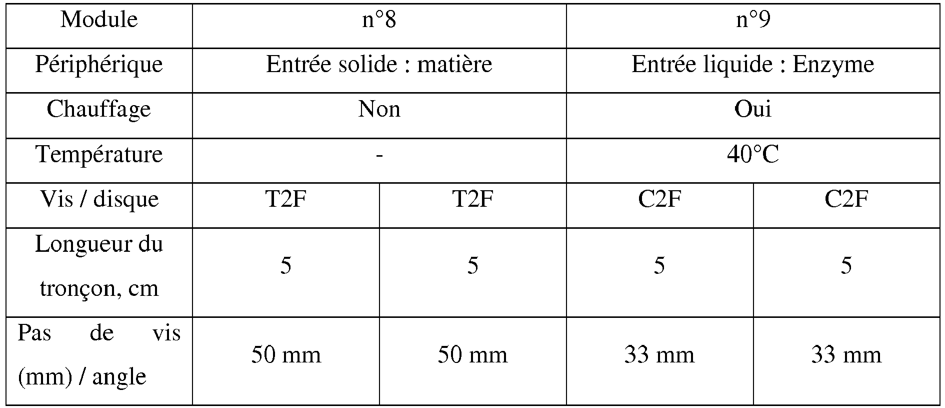

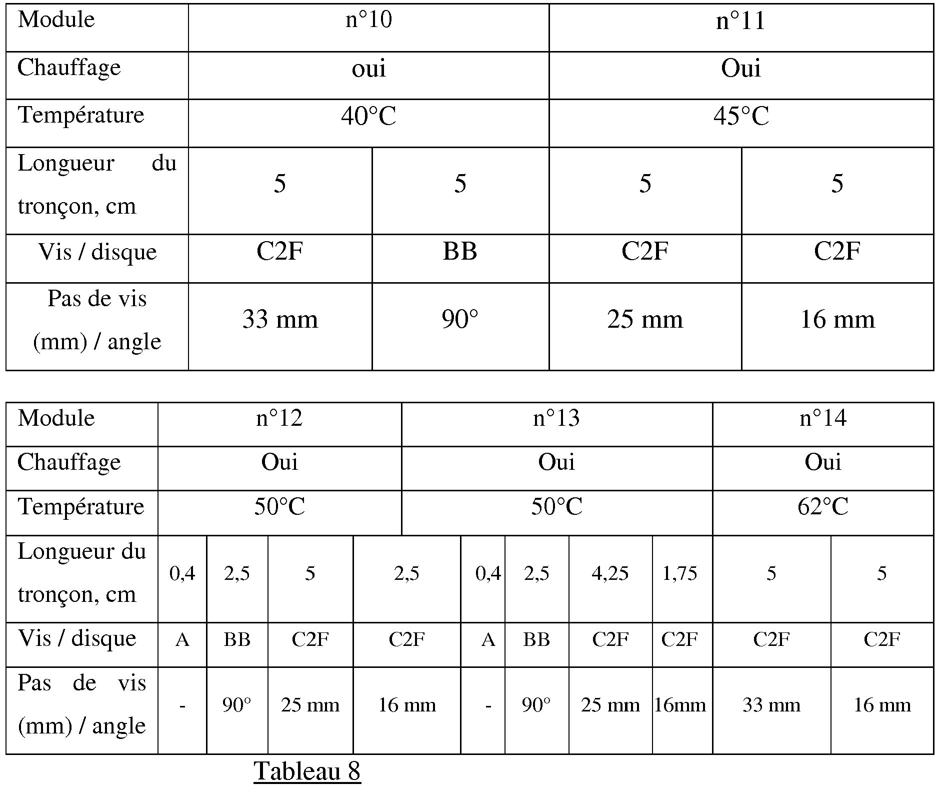

Les conditions de traitement sont décrites dans le tableau 3 ci-dessus. La solution enzymatique utilisée est une solution d'enzymes Sacchariseb C6 (Advanced Enzyme Technologies Ltd) dans un tampon aqueux de citrate à 300 mM présentant une concentration en enzyme de 3 % (m/m).

1 Kg de matière sèche de cette composition bio-extrudée contient 0,92 Kg de matière organique, dont 83g d'enzyme, 0,38 Kg de cellulose, 0,14 Kg d'hémicelluloses, 0,16 Kg de lignines et 0,04 Kg de matière organique hydrosoluble à chaud.

La fermentation de 1 Kg de la composition bio-extrudée (sans ajout d'enzymes supplémentaires) conduit à la formation de 136 g d'éthanol correspondant à 51.5 % du potentiel maximal de fermentation de la totalité des hexoses de la composition bio-extrudée.

### EXEMPLE 3 : Traitement d'une composition de biomasse de paille d'orge.

On collecte une composition de biomasse formée de paille d'orge. On réalise une étape de broyage de la paille d'orge dans un broyeur à marteaux équipé d'une grille de sélection de 5 mm. 1 kg de matière sèche de cette paille d'orge contient 0,93 Kg de matière organique, dont 0,39 Kg de cellulose, 0,26 Kg d'hémicelluloses, 0,16 Kg de lignines et 0,1 Kg de matière organique hydrosoluble à chaud.

### 1) Traitement mécano-chimique

On réalise un premier traitement mécano-chimique de la paille d'orge broyée dans un extrudeur bi-vis CLEXTRAL EV 25 (CLEXTRAL SA, Firminy, France).

La configuration de l'extrudeur est décrite à titre d'exemple non limitatif dans le tableau 9 ci-après, Les conditions opératoires de mise en œuvre sont décrites dans le tableau 10.

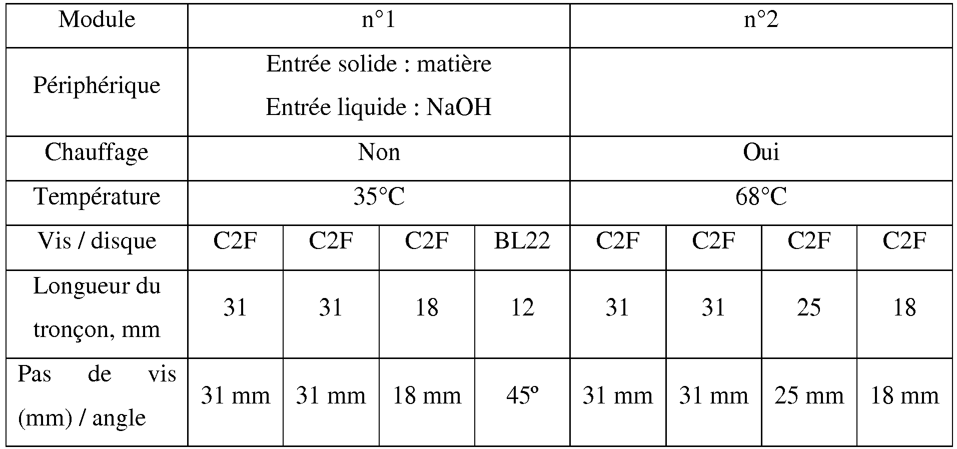

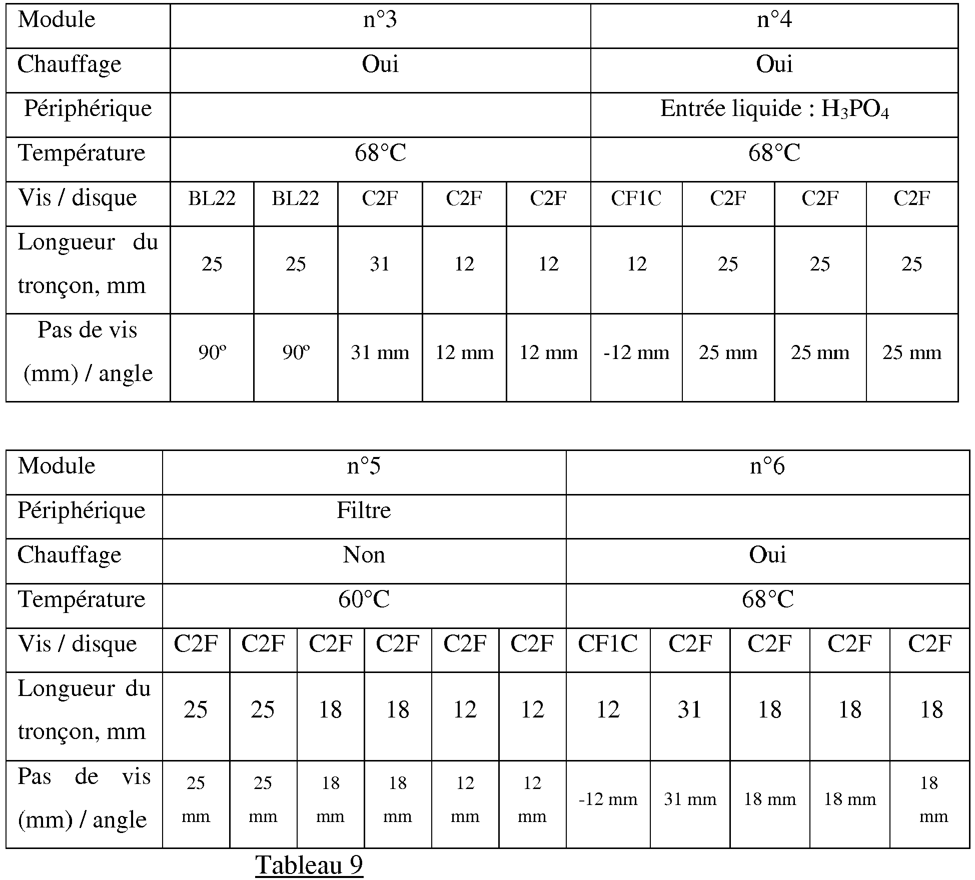

1 Kg de matière sèche de la composition intermédiaire neutralisée contient 0,95 Kg de matière organique, dont 0,07 Kg de matière organique hydrosoluble à chaud.

### 2) Traitement mécano-enzymatique

On reprend cette composition intermédiaire neutralisée et on l'introduit dans un extrudeur bi-vis CLEXTRAL EV 25 pour un traitement mécano-enzymatique.

La configuration de l'extrudeur est décrite dans le tableau 11 ci-après, Les conditions opératoires de mise en œuvre sont décrites dans le tableau 10.

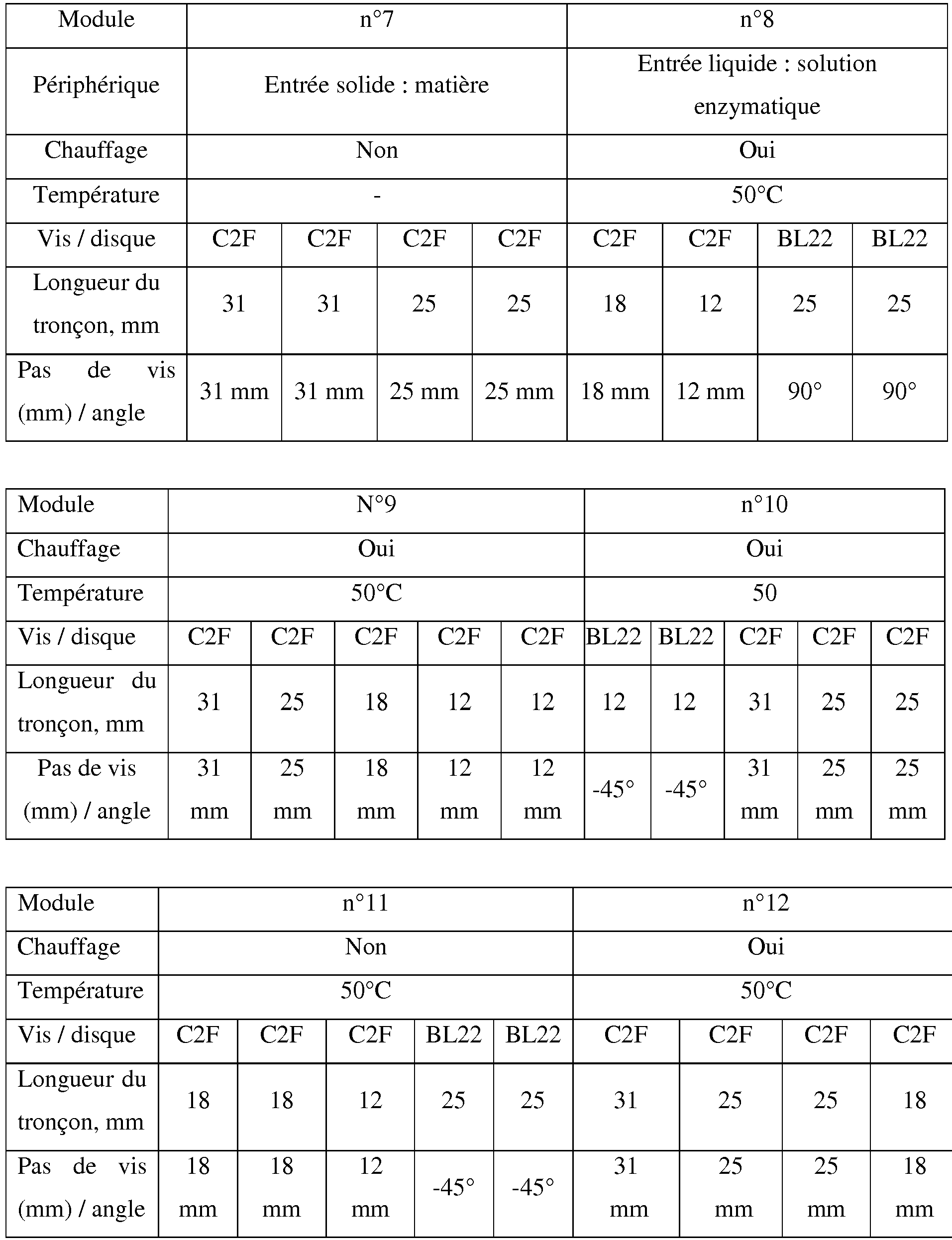

Tableau 11

La solution enzymatique utilisée est une solution d'enzymes (cellulase Cellic CTec2 et xylanase Cellic HTec2 dans une proportion 9 :1 rapporté au contenu en protéines) dans un tampon aqueux de citrate à 200 mM présentant une concentration en enzyme de 2,5 % (masse/masse). 1 Kg de matière sèche de cette composition bio-extrudée contient 0,95 Kg de matière organique, dont 26 g d'enzymes et 0,29 Kg de matière organique hydrosoluble à chaud.

La fermentation de 1kg de la composition bio-extrudée (sans ajout d'enzymes supplémentaires conduit à la formation de 144 g d'éthanol correspondant à 60 % du potentiel maximal de fermentation de la totalité des hexoses de la composition bio-extrudée.

**Tableau 10**

| Traitement | Débit d'entrée de matière sèche (Kg/h) | Vitesse de vis (rpm) | Entrée NaOH | | Entrée H3PO4 | | Solution enzymatique |
|---|---|---|---|---|---|---|---|
| | | | Débit (Kg/h) | Concentration (%(W/V)) | Débit (Kg/h) | Concentration (%(W/V)) | Débit (Kg/h) |
| Mécano-chimique | 0,54 | 150 | 0,34 | 10 | 7,38 | 0,98 | |
| Mécano-enzymatique | 0,2 | 150 | | | | | 0,28 |

### EXEMPLE 4 : Traitement d'une composition de biomasse formée des co-produits industriels de l'agave tequilana.

On collecte une composition de biomasse formée de la bagasse d'agave tequilana Weber var. azul. On réalise une étape de broyage de cette bagasse dans un broyeur à marteaux équipé d'une grille de sélection de 2 mm. 1 Kg de matière sèche de ces résidus de plants d'agave contient 0,96 Kg de matière organique, dont 0,39 Kg de cellulose, 0,17 Kg d'hémicelluloses, 0,19 Kg de lignines et 0,22 Kg de matière organique hydrosoluble à chaud.

On réalise un traitement mécano-chimique et un traitement mécano-enzymatique successifs et en continu de la bagasse broyée successivement dans un extrudeur bi-vis CLEXTRAL Evolum 25 (CLEXTRAL SA, Firminy, France).

La configuration de l'extrudeur bi-vis CLEXTRAL Evolum 25 est décrite à titre d'exemple non limitatif dans le tableau 12 ci-après, les conditions opératoires de mise en œuvre sont décrites dans le tableau 13.

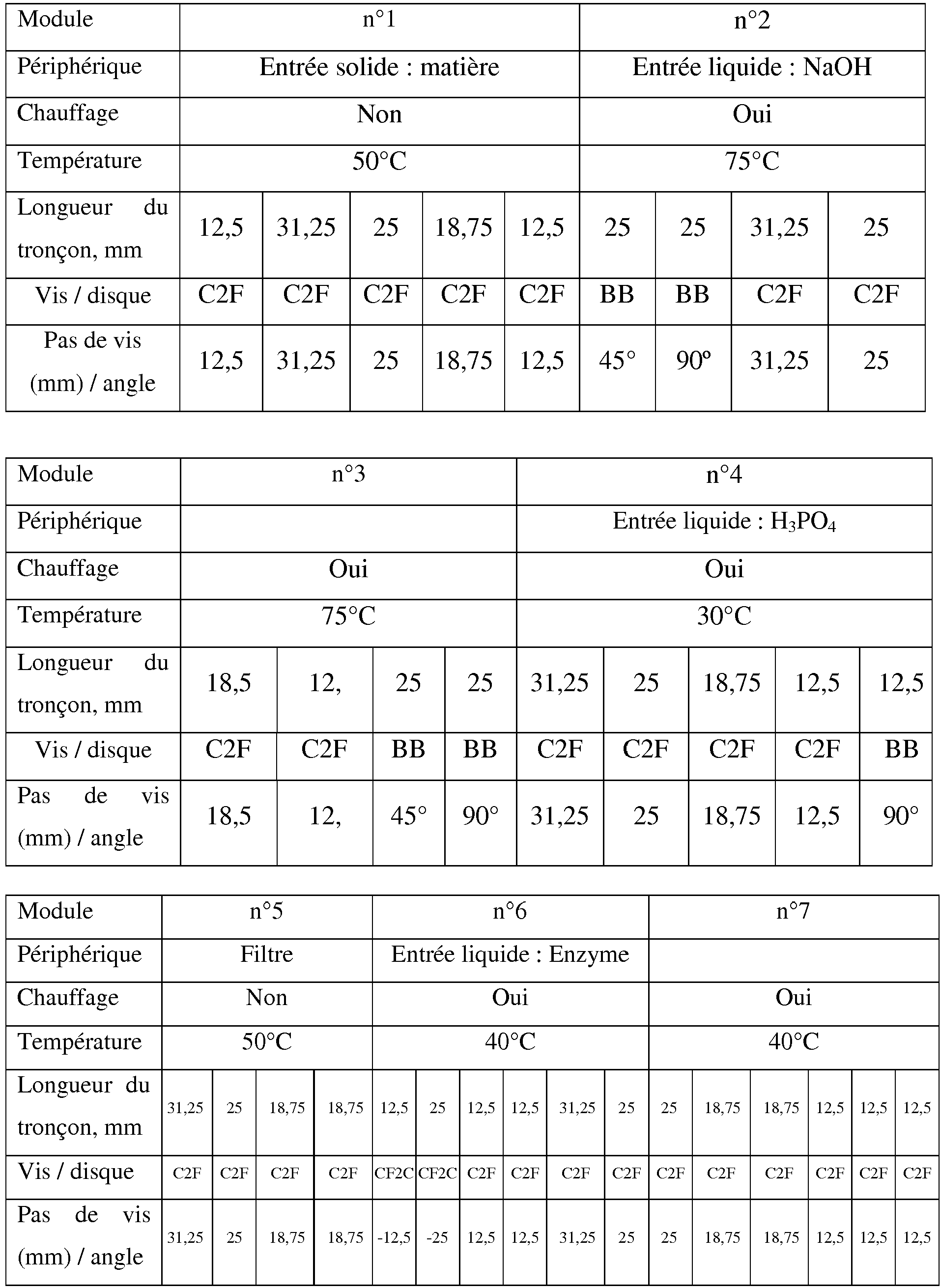

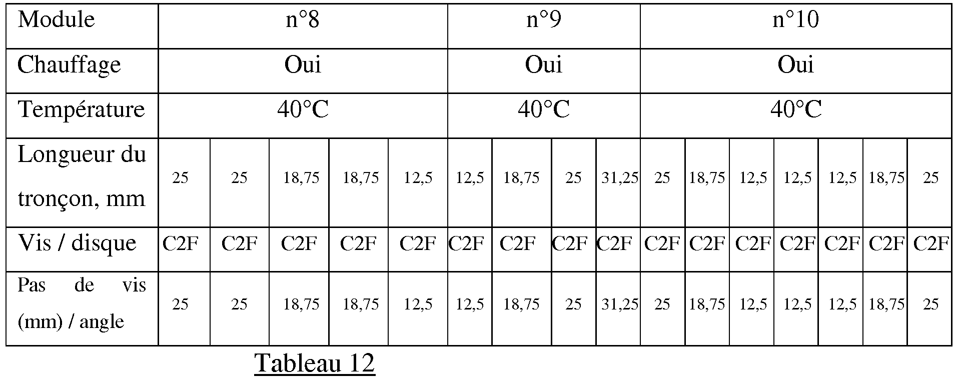

La solution enzymatique utilisée est constituée d'un mélange de Cellic CTec2 (Novozymes) et de Viscozyme (Novozymes) dans un tampon aqueux de citrate à 50 mM présentant une concentration en enzyme de 0,2 % (m/m).

1 kg de matière sèche de cette composition bio-extrudée contient 0,93 Kg de matière organique, dont 9 g d'enzyme, 0,42 Kg de cellulose, 0,10 Kg d'hémicelluloses, 0,16 Kg de lignines et 0,25 Kg de matière organique hydrosoluble à chaud. Dans les conditions opératoires de cet exemple, le traitement de la bagasse permet l'hydrolyse de 22 % de cellulose et 58 % d'hémicelluloses.

La fermentation de 1 Kg de la composition bio-extrudée (sans ajout d'enzymes supplémentaires conduit à la formation de 22,5 g d'éthanol correspondant à 9 % du potentiel maximal de fermentation de la totalité des hexoses de la composition bio-extrudée.

**Tableau 13**

| Traitement | Débit d'entrée de matière sèche (Kg/h) | Vitesse de vis (rpm) | Entrée NaOH | | Entrée H₃PO₄ | | Solution enzymatique |
|---|---|---|---|---|---|---|---|
| | | | Débit (Kg/h) | Concentration (%) | Débit (Kg/h) | Concentration (%) | Débit (Kg/h) |
| Mécano-chimique etmécano-enzymatique | 0,22 | 85 | 0,46 | 5 | 0.395 | 4.78 | 0.46 |

## Revendications

1. Procédé de traitement d'une matière (10) ligno-cellulosique solide dans lequel on soumet ladite matière (10) ligno-cellulosique solide à un traitement, dit traitement (1) mécano-chimique, de malaxage de ladite matière (10) ligno-cellulosique solide, et de dégradation chimique de ladite matière (10) ligno-cellulosique solide, dans lequel on met la matière (10) ligno-cellulosique solide en contact avec une solution alcaline de façon à former une composition intermédiaire présentant une matière ligno-cellulosique, dite matière ligno-cellulosique hydratée, de digestibilité enzymatique augmentée par rapport à la digestibilité de la matière (10) ligno-cellulosique solide de départ, puis ;
on soumet ladite matière ligno-cellulosique hydratée à un traitement, dit traitement (2) mécano-enzymatique, dans lequel on forme une dispersion, dite dispersion aqueuse, de ladite matière ligno-cellulosique hydratée dans une composition aqueuse, ladite dispersion aqueuse comprenant au moins une enzyme de dégradation de ladite matière ligno-cellulosique hydratée, et ;
on réalise le traitement (2) mécano-enzymatique par malaxage de ladite dispersion aqueuse dans un extrudeur bi-vis adapté pour permettre une succession de phases de compression, de détente et de cisaillement mécaniques de la dispersion aqueuse de façon à former une solution, dite solution (36) d'hydrolyse, aqueuse de produits d'hydrolyse de ladite matière (10) ligno-cellulosique solide ;
**caractérisé en ce qu'**on réalise une étape de séparation liquide/solide de ladite solution (36) d'hydrolyse et d'un résidu (40) solide, et **en ce que** ;
on soumet le résidu (40) solide à une fermentation, ce par quoi une quantité d'éthanol est libérée avec un rendement, exprimé en masse d'éthanol produit par fermentation rapportée à la masse du résidu (40) solide, supérieur au rendement de fermentation de la matière (10) ligno-cellulosique solide de départ réalisé dans les mêmes conditions que la fermentation dudit résidu (40) solide

2. Procédé selon la revendication 1, **caractérisé en ce que** la dispersion aqueuse présente un rapport entre la masse de composition aqueuse de la dispersion aqueuse et la masse de matière sèche de ladite matière (10) ligno-cellulosique solide de la dispersion aqueuse compris entre 1 et 4.

3. Procédé selon l'une des revendications 1 ou 2, **caractérisé en ce qu'**on réalise la dispersion aqueuse par addition volontaire d'enzyme(s) de dégradation à la matière (10) ligno-cellulosique solide.

4. Procédé selon l'une des revendications 1 à 3, **caractérisé en ce que** la matière (10) ligno-cellulosique solide comprend :
- une proportion massique de cellulose, exprimée en poids sec de cellulose et en poids sec de la matière (10) ligno-cellulosique solide, comprise entre 20 % et 99 % ;
- une proportion massique d'hémicelluloses, exprimée en poids sec d'hémicelluloses et en poids sec de la matière (10) ligno-cellulosique solide, comprise entre 15 % et 50 % ;
- une proportion massique de lignines, exprimée en poids sec de lignines et en poids sec de la matière (10) ligno-cellulosique solide, comprise entre 0,1 % et 30 %.

5. Procédé selon l'une des revendications 1 à 4, **caractérisé en ce que** la dispersion aqueuse comprend une proportion massique d'enzyme(s) de dégradation par rapport à la matière sèche de la matière (10) ligno-cellulosique solide comprise entre 1 % et 20 %.

6. Procédé selon l'une des revendications 1 à 5, **caractérisé en ce qu'**on réalise le traitement (2) mécano-enzymatique à une température comprise entre 20°C et 80°C.

7. Procédé selon l'une des revendications 1 à 6, **caractérisé en ce qu'**on choisit l'(les) enzyme(s) de dégradation de la matière (10) ligno-cellulosique solide dans le groupe formé des cellulases.

8. Procédé selon l'une des revendications 1 à 7, **caractérisé en ce qu'**on choisit au moins une enzyme de dégradation de la matière (10) ligno-cellulosique solide dans le groupe formé des enzymes de dégradation des lignines.

9. Procédé selon l'une des revendications 1 à 8, **caractérisé en ce qu'**on choisit au moins une enzyme de dégradation de la matière (10) ligno-cellulosique solide dans le groupe formé des enzymes de dégradation des hémicelluloses.

10. Procédé selon l'une des revendications 1 à 9, **caractérisé en ce qu'**on choisit la matière (10) ligno-cellulosique solide dans le groupe formé de tout ou partie d'un plant de maïs, d'une paille de céréale, d'un déchet de fabrication de téquila, de bagasse d'agave pour la production d'inuline, de bagasse de canne à sucre, d'un résidu de production d'huile de palme et d'un tourteau d'une plante oléagineuse.

11. Procédé selon l'une des revendications 1 à 10, **caractérisé en ce qu'**on soumet la solution d'hydrolyse contenant les saccharides solubilisés à une étape de fermentation enzymatique des saccharides solubilisés.

12. Procédé selon l'une des revendications 1 à 11, **caractérisé en ce qu'**on réalise le traitement (1) mécano-chimique à une température comprise entre 50°C et 150°C.

13. Procédé selon l'une des revendications 1 à 12, **caractérisé en ce qu'**on ajoute à la composition intermédiaire un volume d'une solution, dite solution acide, aqueuse d'au moins un acide minéral apte à diminuer le pH de la composition intermédiaire et à former une composition intermédiaire neutralisée apte à être soumise au traitement (2) mécano-enzymatique.

## Patentansprüche

1. Verfahren zur Behandlung eines festen Lignocellulosematerials (10), wobei das feste Lignocellulosematerial (10) einer als mechanischchemischen Behandlung (1) bezeichneten Behandlung des Knetens des festen Lignocellulosematerials (10), und des chemischen Abbaus des festen Lignocellulosematerials (10) unterzogen wird, wobei das feste Lignocellulosematerial (10) mit einer alkalischen Lösung in Kontakt gebracht wird, um eine Zwischenzusammensetzung zu bilden, die ein als hydratisiertes Lignocellulosematerial bezeichnetes Lignocellulosematerial von im Verhältnis zur Verdaulichkeit des anfänglichen festen Lignocellulosematerials (10) erhöhter enzymatischer Verdaulichkeit aufweist, und anschließend;
das hydratisierte Lignocellulosematerial einer als mechanisch-enzymatischen Behandlung (2) bezeichneten Behandlung unterzogen wird, wobei eine als wässrige Dispersion bezeichnete Dispersion des hydratisierten Lignocellulosematerials in einer wässrigen Zusammensetzung gebildet wird, wobei die wässrige Zusammensetzung mindestens ein Abbauenzym des hydratisierten Lignocellulosematerials umfasst, und;
die mechanisch-enzymatische Behandlung (2) durch Kneten der wässrigen Dispersion in einem Zweischneckenextruder ausgeführt wird, der dafür geeignet ist, eine Abfolge von mechanischen Verdichtungs-, Entspannungs- und Scherphasen der wässrigen Dispersion zu ermöglichen, um eine als Hydrolyselösung (36) bezeichnete wässrige Lösung von Hydrolyseprodukten des festen Lignocellulosematerials (10) zu bilden;
**dadurch gekennzeichnet, dass** ein Schritt der Fest-Flüssig-Trennung der Hydrolyselösung (36) und eines festen Rückstands (40) ausgeführt wird, und dadurch, dass;
der feste Rückstand (40) einer Fermentation unterzogen wird, wodurch eine Menge an Ethanol freigesetzt wird mit einer Ausbeute, ausgedrückt als fermentativ erzeugte Ethanolmasse im Verhältnis zur Masse des festen Rückstands (40), die höher ist als die Ausbeute aus Fermentation des anfänglichen festen Lignocellulosematerials (10), welche unter den gleichen Bedingungen ausgeführt wird wie die Fermentation des festen Rückstands (40).

2. Verfahren nach Anspruch 1, **dadurch gekennzeichnet, dass** die wässrige Dispersion ein Verhältnis zwischen der Masse wässriger Zusammensetzung der wässrigen Dispersion und der Trockenmasse des festen Lignocellulosematerials (10) der wässrigen Dispersion im Bereich zwischen 1 und 4 aufweist.

3. Verfahren nach einem der Ansprüche 1 oder 2, **dadurch gekennzeichnet, dass** die wässrige Dispersion durch optionale Zugabe von Abbauenzym(en) zum festen Lignocellulosematerial (10) hergestellt wird.

4. Verfahren nach einem der Ansprüche 1 bis 3, **dadurch gekennzeichnet, dass** das feste Lignocellulosematerial (10) umfasst:
- einen Massenanteil an Cellulose, ausgedrückt als Trockengewicht Cellulose und als Trockengewicht des festen Lignocellulosematerials (10), im Bereich zwischen 20 % und 99 %;
- einen Massenanteil an Hemicellulosen, ausgedrückt als Trockengewicht Hemicellulosen und als Trockengewicht des festen Lignocellulosematerials (10), im Bereich zwischen 15 % und 50 %;
- einen Massenanteil an Ligninen, ausgedrückt als Trockengewicht Lignine und als Trockengewicht des festen Lignocellulosematerials (10), im Bereich zwischen 0,1 % und 30 %.

5. Verfahren nach einem der Ansprüche 1 bis 4, **dadurch gekennzeichnet, dass** die wässrige Dispersion einen Massenanteil an Abbauenzym(en) im Verhältnis zur Trockenmasse des festen Lignocellulosematerials (10) im Bereich zwischen 1 % und 20 % umfasst.

6. Verfahren nach einem der Ansprüche 1 bis 5, **dadurch gekennzeichnet, dass** die mechanisch-enzymatische Behandlung (2) bei einer Temperatur im Bereich zwischen 20 °C und 80 °C ausgeführt wird.

7. Verfahren nach einem der Ansprüche 1 bis 6, **dadurch gekennzeichnet, dass** das/die Abbauenzym(e) des festen Lignocellulosematerials (10) aus der Gruppe gewählt werden, die von den Cellulasen gebildet wird.

8. Verfahren nach einem der Ansprüche 1 bis 7, **dadurch gekennzeichnet, dass** mindestens ein Abbauenzym des festen Lignocellulosematerials (10) aus der Gruppe gewählt wird, die von den Abbauenzymen der Lignine gebildet wird.

9. Verfahren nach einem der Ansprüche 1 bis 8, **dadurch gekennzeichnet, dass** mindestens ein Abbauenzym des festen Lignocellulosematerials (10) aus der Gruppe gewählt wird, die von den Abbauenzymen der Hemicellulosen gebildet wird.

10. Verfahren nach einem der Ansprüche 1 bis 9, **dadurch gekennzeichnet, dass** das feste Lignocellulosematerial (10) aus der Gruppe gewählt wird, die von der ganzen oder einem Teil einer Maispflanze, einem Getreidestroh, einem Abfall aus der Tequila-Herstellung, Agaven-Bagasse für die Produktion von Inulin, Zuckerrohr-Bagasse, einem Rückstand aus der Produktion von Palmöl und einem Presskuchen einer Ölpflanze gebildet wird.

11. Verfahren nach einem der Ansprüche 1 bis 10, **dadurch gekennzeichnet, dass** die Hydrolyselösung, die die solubilisierten Saccharide enthält, einem Schritt der enzymatischen Fermentation der solubilisierten Saccharide unterzogen wird

12. Verfahren nach einem der Ansprüche 1 bis 11, **dadurch gekennzeichnet, dass** die mechanisch-chemische Behandlung (1) bei einer Temperatur im Bereich zwischen 50 °C und 150 °C ausgeführt wird.

13. Verfahren nach einem der Ansprüche 1 bis 12, **dadurch gekennzeichnet, dass** der Zwischenzusammensetzung ein Volumen einer als saure Lösung bezeichneten wässrigen Lösung mindestens einer anorganischen Säure zugegeben wird, die den pH-Wert der Zwischenzusammensetzung senken und eine neutralisierte Zwischenzusammensetzung bilden kann, welche der mechanisch-enzymatischen Behandlung (2) unterzogen werden kann.

## Claims

1. A method of treatment of a solid lignocellulosic material (10) in which said solid lignocellulosic material (10) is subjected to a treatment, called mechano-chemical treatment (1), of kneading of said solid lignocellulosic material (10) and chemical degradation of said solid lignocellulosic material (10), in which the solid lignocellulosic material (10) is brought into contact with an alkaline solution in a manner such as to form an intermediate composition comprising a solid lignocellulosic material, called hydrated lignocellulosic material, having an enzymatic digestibility which is increased with respect to the digestibility of the starting solid lignocellulosic material (10); and then
the hydrated lignocellulosic material is subjected to a treatment, called mechano-enzymatic treatment (2), in which a dispersion, called aqueous dispersion, of the hydrated lignocellulosic material (10) in an aqueous composition is formed, said aqueous dispersion comprising at least one enzyme for degradation of said hydrated lignocellulosic material; and
the mechano-enzymatic treatment (2) is carried out by kneading said aqueous dispersion in a kneading reactor suitable for allowing a succession of mechanical phases of compression, expansion and shearing of the aqueous dispersion in a manner such as to form an aqueous solution, called hydrolysis solution (36), of hydrolysis products of said solid lignocellulosic material (10);
wherein a liquid/solid separation step is carried out between said hydrolysis solution and a solid residue, and wherein;
the solid residue is subjected to fermentation, whereby an amount of ethanol is released with a yield, expressed as the mass of ethanol produced by fermentation relative to the mass of the solid residue, higher than the fermentation yield of the starting solid ligno-cellulosic material (10) under the same conditions as the fermentation of said solid residue.

2. A method as claimed in claim 1, wherein the aqueous dispersion has a ratio between the weight of the aqueous composition of the aqueous dispersion and the weight of the dry matter of said solid lignocellulosic material (10) of the aqueous dispersion of between 1 and 4.

3. A method as claimed in one of claims 1 or 2, wherein the aqueous dispersion is obtained by optional addition of degradation enzyme(s) to the solid lignocellulosic material (10) .

4. A method as claimed in one of claims 1 to 3, wherein the solid lignocellulosic material (10) comprises:
- a proportion by weight of cellulose, expressed in dry weight of cellulose and in dry weight of the solid lignocellulosic material (10), of between 20 % and 99 %;
- a proportion by weight of hemicelluloses, expressed in dry weight of hemicelluloses and dry weight of the solid lignocellulosic material (10), of between 15 % and 50 %;
- a proportion by weight of lignins, expressed in dry weight of lignins and dry weight of the solid lignocellulosic material (10), of between 0.1 % and 30 %;

5. A method as claimed in one of claims 1 to 4, wherein the aqueous dispersion comprises a proportion by weight of degradation enzyme(s) with respect to the dry matter of the solid lignocellulosic material (10) of between 1 % and 20 %.

6. A method as claimed in one of claims 1 to 5, wherein the mechano-enzymatic treatment (2) is carried out at a temperature of between 20 °C and 80 °C.

7. A method as claimed in one of claims 1 to 6, wherein the enzyme(s) for degradation of the solid lignocellulosic material (10) is/are selected from the group formed by cellulases.

8. A method as claimed in one of claims 1 to 7, wherein at least one enzyme for degradation of the solid lignocellulosic material (10) is selected from the group formed by enzymes for degradation of lignins.

9. A method as claimed in one of claims 1 to 8, wherein at least one enzyme for degradation of the solid lignocellulosic material (10) is selected from the group formed by enzymes for degradation of hemicelluloses.

10. A method as claimed in one of claims 1 to 9, wherein the solid lignocellulosic material (10) is selected from the group formed by all or part of a maize plant, cereal straw, waste from the production of tequila, agave bagasse for the production of inulin, sugar-cane bagasse, a residue from the production of palm oil and a cake of an oleaginous plant.

11. A method as claimed in one of claims 1 to 10, wherein the hydrolysis solution comprising solubilized saccharides is subjected to a step of enzymatic fermentation of the solubilized saccharides.

12. A method as claimed in one of claims 1 to 11, wherein the mechano-chemical treatment (1) is carried out at a temperature of between 50 °C and 150 °C.

13. A method as claimed in one of claims 1 to 12, wherein a volume of an aqueous solution, called acid solution, of at least one mineral acid suitable for reducing the pH of the intermediate composition and for the formation of a neutralized intermediate composition suitable for being subjected to the mechano-enzymatic treatment (2) is added to the intermediate composition.
